(19) Europäisches Patentamt
European Patent Office
Office européen des brevets

(11) **EP 4 597 505 A1**

(12) **EUROPEAN PATENT APPLICATION**
published in accordance with Art. 153(4) EPC

(43) Date of publication:
06.08.2025 Bulletin 2025/32

(21) Application number: 23874506.1

(22) Date of filing: 28.07.2023

(51) International Patent Classification (IPC):
*G16C 10/00* (2019.01)     *G06N 10/40* (2022.01)
*G06N 10/80* (2022.01)     *G06N 99/00* (2019.01)

(52) Cooperative Patent Classification (CPC):
G06N 10/40; G06N 10/80; G06N 99/00;
G16C 10/00

(86) International application number:
PCT/JP2023/027741

(87) International publication number:
WO 2024/075373 (11.04.2024 Gazette 2024/15)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 04.10.2022 JP 2022160169
19.12.2022 JP 2022202115
16.01.2023 JP 2023004739

(71) Applicants:
• Quemix Inc.
Tokyo 1030027 (JP)
• INSTITUTE OF SCIENCE TOKYO
Tokyo 152-8550 (JP)

(72) Inventors:
• KOSUGI, Taichi
Tokyo 103-0027 (JP)
• NISHI, Hirofumi
Tokyo 103-0027 (JP)
• NISHIYA, Yusuke
Tokyo 103-0027 (JP)
• COUZINIE, Yannick
Tokyo 103-0027 (JP)
• TRAN, Hung Ba
Tokyo 103-0027 (JP)
• MATSUSHITA, Yuichiro
Tokyo 152-8550 (JP)

(74) Representative: Winter, Brandl - Partnerschaft
mbB
Alois-Steinecker-Straße 22
85354 Freising (DE)

(54) **INFORMATION PROCESSING SYSTEM, INFORMATION PROCESSING METHOD, AND INFORMATION PROCESSING PROGRAM**

(57) According to one aspect of the present invention, an information processing system is provided. The information processing system comprises at least one processor capable of executing a program so as to carry out each of the following steps. An acquisition step involves performing a process of acquiring substance information relating to a substance including at least one atomic nucleus and at least one electron. The substance information includes: a plurality of disposition candidates for the atomic nucleus; and interactions that act on the atomic nucleus and the electron. The interactions at least include an electron-atomic nucleus interaction that acts between the atomic nucleus and the electron. An allocation step involves performing a process of allocating each of the acquired disposition candidates for the atomic nucleus to one of one or more first quantum bit states. The first quantum bit states are configured by one or more first quantum bits and can be observed through observa-

tion operation on the first quantum bits. A superposition step involves performing a process of generating a superposition state of a plurality of the first quantum bit states, by performing a prescribed first quantum gate operation on the first quantum bits. An interaction step involves performing a process of, by performing a second quantum gate operation, including the acquired interactions, on the first quantum bits representing the superposition state and on one or more second quantum bits representing the state of the electron of the substance, generating a correlated quantum bit state in which the first quantum bits and the second quantum bits are correlated with each other. An identification step involves performing a process of identifying, on the basis of a result of observation on the first quantum bits in the generated correlated quantum bit state, a disposition where the minimum energy eigenvalue is relatively low, from among the disposition candidates for the atomic

EP 4 597 505 A1

nucleus.

FIG. 1

## Description

### BACKGROUND

The present invention relates to an information processing system, an information processing method, and an information processing program.

### RELATED ART

In the patent document 1, a molecular dynamics simulation apparatus is disclosed, characterized by having a coordinate information storage unit that stores information on nuclear coordinates and internal coordinates, a potential constant information storage unit that takes different values depending on the coordination state of each atomic molecule, a dynamic bond order evaluation unit that calculates bond orders between atoms based on the nuclear coordinates and internal coordinates, a coordination state determination unit that determines the coordination state of atoms based on the bond orders, a potential energy calculation unit that calculates the total potential energy based on the nuclear coordinates, the internal coordinates, the bond orders, and the coordination state, a first derivative calculation unit that calculates the force acting on each nuclear coordinate and internal coordinate by taking the first derivative of the potential energy, a simulation unit that performs molecular dynamics calculations on the time evolution of the nuclear coordinates and the internal degree of freedom variables while repeating the above-mentioned calculations a specified number of times, and an output unit that outputs the results of the simulations.

By applying the technology described in the patent document 1, the structural energy change dependent on the coordination state of each atom can be faithfully reproduced in molecular dynamics simulations of large-scale systems that handle an ensemble of several thousand or more atoms, while efficiently reproducing the structural changes that accompany the recombination of covalent bonds in substances with a complex composition in which multiple elements are mixed.

### PRIOR ART DOCUMENTS

Patent document

[Patent Document 1] JP 2008-052308 A

### SUMMARY

Problems to be Solved by Invention

By the way, when simulations are performed for a substance, the arrangement of the particles that make up the substance can be important. Therefore, it is desirable to obtain the arrangement of particles with a high degree of accuracy. However, when there are multiple candidates for the arrangement of particles, it is necessary to perform dynamics simulations for each of the multiple candidate particle arrangements sequentially, which may increase the computation time.

Means for Solving Problems

According to one aspect of the present invention, an information processing system is provided. This information processing system comprises at least one processor configured to execute a program so as to perform each of the following steps. An acquisition step is the process of acquiring material information about a substance that contains at least one nucleus and at least one electron. The substance information includes a plurality of configuration candidates of the nuclei configuration candidate and the interactions that act on each of the nucleus and the electron. The interactions include at least the electron-nucleus interaction, which acts between the nucleus and the electron. An assignment step assigns each of the acquired configuration candidates of the nuclei to at least one first qubit state. The first qubit state is composed of at least one first qubit and is observable by an observation operation on the first qubit. A superposition step is the process of generating a superposition state of multiple first qubit states by performing a predetermined first quantum gate operation on the first qubit. An interaction step is to perform a second quantum gate operation, including the acquired interaction, on the first qubit representing the superposition state and at least one second qubit representing the electronic state of matter, to produce a correlated qubit state in which the first and second qubits are correlated with each other. An identifying step identifies a configuration with relatively low minimum energy eigenvalue among the candidate configurations of nuclei based on the observation results for the first qubit in the generated correlated qubit state.

According to such an information processing system, it is possible to improve the computational efficiency in identifying an appropriate particle configuration from among multiple candidate particle configurations.

**BRIEF DESCRIPTION OF DRAWINGS**

[FIG. 1] Fig. 1is a diagram representing the information processing system 1.

[FIG. 2] Fig. 2 is a block diagram showing the hardware configuration of the information processor apparatus 2.

[FIG. 3] Fig. 3is a block diagram showing the hardware configuration of the quantum computer 3.

[FIG. 4] Fig. 4 is a block diagram showing the hardware configuration of user terminal 4.

[FIG. 5] Fig. 5 is a block diagram showing the functional configuration of processor 23.

[FIG. 6] Fig. 6 is an activity diagram that shows an overview of the information processing performed in the information processing system 1.

[FIG. 7] Fig. 7 shows an example of a computational quantum circuit QC1.

[FIG. 8] Fig. 8 illustrates an example of the first quantum gate operation QC11.

[FIG. 9] Fig. 9 illustrates an example of the reference quantum gate operation QC12.

[FIG. 10] Fig. 10 illustrates the computational quantum circuit QC1 based on the probabilistic imaginary-time evolution method.

[FIG. 11] Fig. 11 shows an example of the representation of the contribution $\exp(-iV\Delta t)$ of the interaction V within the unit quantum circuit QC131.

[FIG. 12] Fig. 12 shows an example of a quantum circuit for implementing the real-time evolution operator $\exp(-iV\_en\Delta t)$ in the case $n\_e=4$ and $n\_nucl=3$.

[FIG. 13] Fig. 13 shows an example of the search quantum circuit C_PITE for imaginary time development.

[FIG. 14] Fig. 14 shows an example of a quantum amplitude amplification circuit Q in the imaginary-time evolution method.

[FIG. 15] Fig. 15 shows the simulation results of the first step (i.e., the initial state).

[FIG. 16] Fig. 16 shows the simulation results of the eighth step.

[FIG. 17] Fig. 17 shows the simulation results of the 14th step.

[FIG. 18] Fig. 18 shows the simulation results of the weights of each candidate structure just after 19 steps have elapsed.

[FIG. 19] Fig. 19 is an activity diagram that shows an overview of the information processing in this form that is performed in the information processing system 1.

[FIG. 20] Fig. 20 is an example of a computational quantum circuit QC1 for VQE.

[FIG. 21] Fig. 21 shows the differential circuit QC2 for computing the derivative $(-2i)$-a$|\varphi(\theta)>/\partial\theta\_k$ with respect to $\theta\_k$.

[FIG. 22] Fig. 22 shows the simulation results of the sum of the weights of the energy eigenstates for each nuclear configuration (Geom 0 to Geom 7).

[FIG. 23] Fig. 23 shows the weights of the ground state (1st lowest), the first excited state (2nd lowest), the second excited state (3rd lowest), and the sum (Total) energy eigenstates of the electronic wave function in the optimal configuration (Geom 4).

[FIG. 24] Fig. 24 shows an example of a unit quantum circuit QC131 for information processing methods for a qubit system that considers only the nuclear configuration.

[FIG.25] Fig. 25 shows an example of the second quantum gate operation QC13 that generates Gibbs states for performing finite temperature calculations.

[FIG. 26] Fig. 26 shows an example of the maximally entangled state generator circuit U_ME.

[FIG. 27] Fig. 27 is a flowchart of the quantum circuit generation process in the present embodiment.

[FIG. 28] Fig. 28 shows an example of the input generation circuit QC10 in the present embodiment.

[FIG. 29] Fig. 29 shows an example of a quantum circuit representing a virtual Hamiltonian as the second quantum gate operation QC13.

[FIG. 30] Fig. 30 shows an example of the computational quantum circuit QC1 in the present embodiment.

[FIG. 31] Fig. 31 shows the relationship between the observation probability of each candidate and the number of steps as a simulation result when the adiabatic time evolution method is employed as the ground state search method.

[FIG. 32] Fig. 32 shows the components in the wave function at the final state.

[FIG. 33] Fig. 33 is a flowchart showing the fourth information processing flow.

[FIG. 34] Fig. 34 is a flowchart showing the fifth information processing flow.

## DETAILED DESCRIPTION

The following describes the embodiment of the present invention using the above diagrams. The features shown in the following embodiments can be combined with each other.

By the way, the program to realize the software appearing in the present embodiment may be provided as a non-transitory computer-readable medium. It may also be provided downloadable from an external server, or it may be provided so that the program can be activated on an external computer to realize its functions on a client terminal (so-called cloud computing).

The term "unit" in the present embodiment may also include, for example, hardware resources implemented by circuitry in the broad sense, together with software information processing that can be specifically realized by these hardware resources. In addition, various types of information are handled in the present embodiment, which are represented, for example, by physical signal values representing voltage or current, high and low signal values as a binary bit set consisting of 0s or 1s, or quantum superpositions (so-called qubits), and communication and computation can be performed on a circuitry in the broad sense.

A circuitry in the broad sense is a circuitry realized by at least an appropriate combination of circuits, circuitries, processors, and memory. This includes Application Specific Integrated Circuits (ASICs), programmable logic devices (e.g., Simple Programmable Logic Devices (SPLDs), Complex Programmable Logic Devices (CPLDs), and Field Programmable Gate Arrays (FPGAs)), etc.

Hardware configuration

This section describes the hardware configuration of the information processing system 1.

<Information processing system 1>

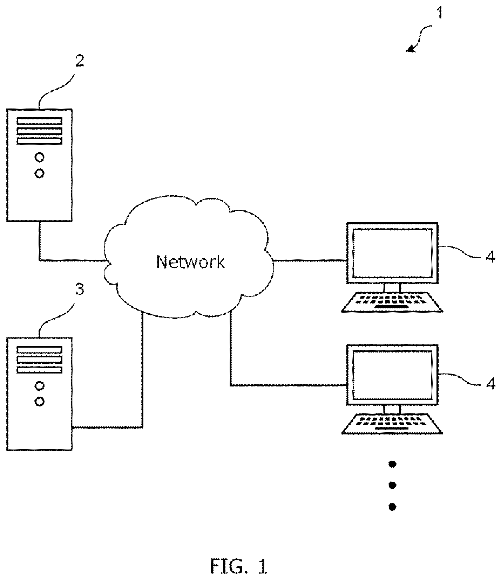

FIG. 1 is a diagram of the information processing system 1. The information processing system 1 is equipped with an information processing apparatus 2, at least one quantum computer 3, and a user terminal 4. The information processing apparatus 2, the quantum computer 3, and the user terminal 4 are configured to communicate through telecommunication lines. In a particular embodiment, an information processing system 1 is one or more pieces of apparatus or components. If, for example, the information processing system 1 consists only of information processing apparatus 2, then the information processing system 1 could be information processing apparatus 2. These components are described below.

<Information processing apparatus 2>

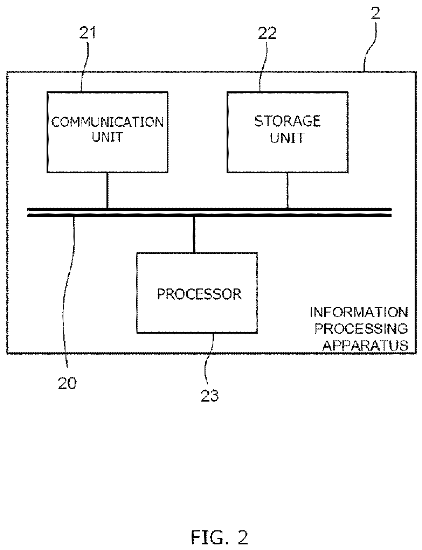

FIG. 2 is a block diagram showing the hardware configuration of the information processing apparatus 2. The information processing apparatus 2 has a communication unit 21, a storage unit 22, and a processor 23, and these components are electrically connected inside the information processing apparatus 2 via the communication bus 20. Each component is further described below.

<communication unit 21>

For the communication unit 21, although wired type communication is preferred, such as USB, IEEE1394, Thunderbolt (registered trademark), wired LAN network communication, etc., wireless LAN network communication, mobile communication such as 3G/LTE/5G, BLUETOOTH (registered trademark) communications, etc. may be included as necessary. In other words, it is preferable to implement it as a set of these multiple communication methods. In other words, information processing apparatus 2 may communicate various information from the outside through the communication unit 21 and network.

<storage unit 22>

The storage unit 22 stores various information as defined in the aforementioned descriptions. This can be implemented, for example, as a storage device such as a solid state drive(SSD) that stores various programs, etc. pertaining to the information processing apparatus 2 executed by the processor 23, or as a memory such as random access memory (RAM) that stores temporarily necessary information (arguments, arrays, etc.) pertaining to program operations. The memory unit 22 stores various programs, variables, etc. pertaining to the information processing apparatus 2 executed by the processor 23.

<Processor 23>

The processor 23 processes and controls the overall operation related to the information processing apparatus 2. The processor 23 is, for example, an unshown central processing unit (CPU). The processor 23 realizes various functions pertaining to the information processing apparatus 2 by reading the predetermined program stored in the memory unit 22. In other words, information processing by software stored in the storage unit 22 is specifically realized by the processor 23, which is an example of hardware, and can be executed as each functional part included in the processor 23. These are described in more detail in the next section. The system is not limited to having a single processor 23 but may be implemented to have multiple processors 23 for each function. It may also be a combination of these.

<Quantum Computer 3>

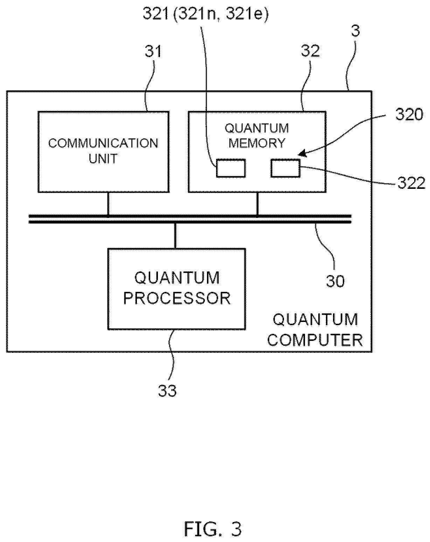

FIG. 3 is a block diagram showing the hardware configuration of the quantum computer 3. As shown in FIG. 3, the quantum computer 3 has a communication unit 31, a quantum memory 32, and a quantum processor 33, and these components are connected inside the quantum computer 3 via a communication bus 30. The quantum computer 3 may include an fault tolerant quantum computer, a NISQ device, or both. The quantum computer 3 in this embodiment is a gate-type. Each component is further described below.

<communication unit 31>

The communication unit 31 is used by the quantum computer 3 to communicate information with other information processing apparatus (including classical computers, quantum computers or combinations thereof) or peripheral devices

<Quantum memory32>

The quantum memory 32 stores various information as defined in the foregoing description. In particular, the quantum memory 32 stores various programs that can be read by the quantum processor 33, which is described next. The quantum memory 32 also stores information on the physical properties of specific materials and other information pertaining to the calculations by the quantum computer 3 as necessary. The quantum memory 32 is equipped with a plurality of the qubits 320. The qubits 320 can be implemented in any way, including nuclear spins, photons, ions, atoms, quantum dots, and superconducting Josephson devices. The qubits 320 include the computational qubits 321 and the ancillary bits 322. The computational qubits 321 includes at least one first qubit 321n and at least one second qubit 321e. The first qubits 321n function as qubits for representing the configuration of atomic nuclei in a material. The second qubit 321e functions as a qubit to represent the electronic state of matter. In detail, the second qubits 321e function as qubits that represent, for example, the configuration of electrons in a material. The quantum memory 32 may include classical memory devices.

<Quantum Processor 33>

The quantum processor 33 processes and controls the overall operation related to the quantum computer 3. The quantum processor 33 realizes various functions related to the quantum computer 3 by reading the program stored in the quantum memory 32 or the predetermined program that is input via the communication unit 31. In FIG. 3, it is represented as a single quantum processor 33, but it may be implemented to have multiple quantum processors 33 for each function. It may also be a combination of these.

The quantum processor 33 is configured to perform various quantum operations on the qubits 320 that can be implemented on a quantum circuit. For example, a quantum circuit is configured to specify a series of quantum operations on the qubits 320. Quantum operations include, for example, quantum gate operations and observation operations. Quantum gate operations correspond to unitary operations on the quantum state of the qubits 320. Observation operations correspond to projection operations on the quantum state of the qubits 320.

<User terminal 4>

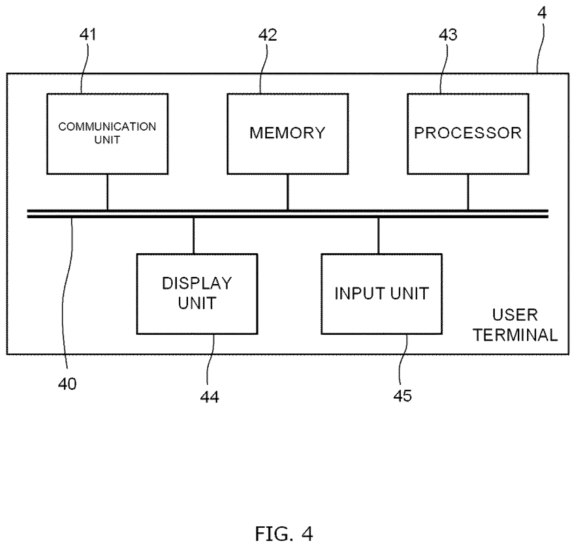

Next, the hardware configuration of user terminal 4 is described. FIG. 4 is a block diagram showing the hardware configuration of user terminal 4. User terminal 4 has a communication unit 41, a memory 42, a processor 43, a display unit 44, input unit 45, and these components are electrically connected inside the user terminal 4 via the communication bus 40. The description of the communication unit 41, the memory 42 and the processor 43 is the same as the description of each part in the information processing apparatus 2 and is therefore omitted.

<display unit 44>

The display unit 44 may be included in the housing of the user terminal 4 or may be external. The display unit 44 displays a graphical user interface (GUI) screen that can be operated by the user. This should be done, for example, by using different display devices such as CRT displays, liquid crystal displays, organic EL displays and plasma displays, depending on the type of the user terminal 4.

<input unit 45>

The input unit 45 may be included in the housing of the user terminal 4 or may be external. For example, the input unit 45 may be integrated with the display unit 44 and implemented as a touch-panel. With a touch panel, the user can input taps, swipes, etc. Of course, a switch button, mouse, QWERTY keyboard, etc. may be employed instead of a touch-panel. That is, the input unit 45 accepts operational input made by the user. The input is transferred as an instruction signal to the processor 43 via the communication bus 40, and the processor 43 can perform predetermined controls and calculations as necessary.

2. Functional configuration of processor 23

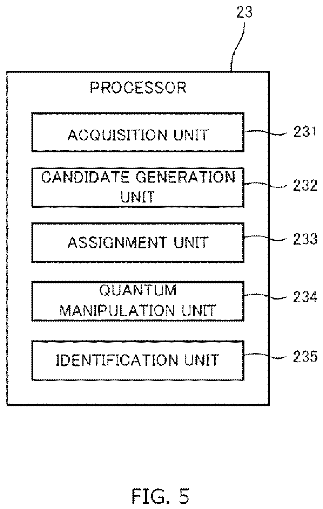

This section describes a functional configuration of the processor 23 of the information processing apparatus 2. FIG. 5 is a block diagram showing the functional structure of the processor 23. The processor 23 has an acquisition unit 231, a candidate generation unit 232, an assignment unit 233, a quantum manipulation unit 234, and an identification unit 235.

<Acquisition unit 231>

The acquisition unit 231 is configured to acquire various information on quantum computation from the quantum computer 3 and the user terminal 4. The acquisition unit 231 is configured to acquire various information by reading various information stored in the storage area, which is at least a part of the storage unit 22, and writing the read information into the work area, which is at least a part of the storage unit 22. The storage area is, for example, an area of the storage unit 22 that is implemented as a storage device such as SSD. The work area is an area that is implemented as a memory, for example, RAM.

<Candidate generator 232>

The candidate generator 232 generates a variety of information about quantum computation. For example, the candidate generator 232 generates configuration candidates of the nuclei based on the information obtained by the acquisition unit 231.

<assignment unit 233>

The assignment unit 233 is configured to be able to assign various information, e.g., configuration candidates of the nuclei, to the states of the qubits 320.

<Quantum manipulation unit 234>

The quantum manipulation unit 234 is configured to perform various quantum operations on the qubits 320. The quantum manipulation unit 234 may be configured to directly perform quantum operations on the qubits 320 or to send a command to the quantum processor 33 to perform quantum operations. The quantum manipulation unit 234 in this implementation sends various quantum circuits to the quantum processor 33, causing the quantum processor 33 to perform quantum operations.

<Identification unit 235>

The identification unit 235 is configured to be able to identify various types of information based on the results of quantum calculations using the qubits 320. For example, based on the results of the quantum calculation, the identification unit 235 identifies the optimal configuration of the nuclei under the given conditions from among the generated configuration candidates of the nuclei.

3. information processing methods

This chapter describes the flow of information processing performed in the information processing system 1 described above. The information processing described in this chapter is sometimes denoted as the first information processing.

3.1. Information processing flow

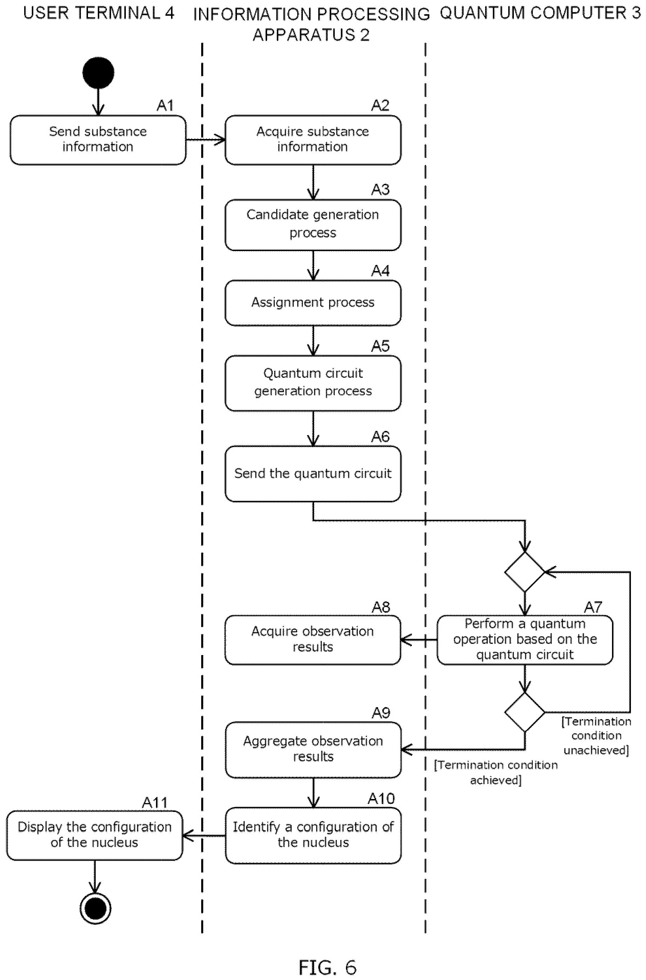

FIG. 6 is an activity diagram showing an overview of the information processing performed in the information processing system 1. Note that the information processing may include any exception processing that is not illustrated in the flowchart. Exception processing includes interruption of the information processing and omission of each processing. Selection or input performed in the information processing may be based on user operation or may be performed automatically without user operation.

In this information processing, the quantum processor 33 initializes the qubits 320 as necessary. If the qubits 320 take two states, the ground state and the excited state, for example, the quantum processor 33 will put all qubits 320 in the ground state. Hereinafter, for convenience of explanation, the ground state and the excited state of a single qubit 320 may be denoted as |0> and |1>, respectively, using bracket notation.

The information processing method can be used, for example, to identify the optimal configuration of the nuclei in a given material. In the following description, the natural unit system is adopted, but other unit systems, such as the SI unit system, CGS unit system, etc., can be adopted arbitrarily.

[Activity A1.]

First, at activity A1, user terminal 4 sends information about the substance to processor 23. Such information includes information that can identify the substance, such as the name of the substance. Such information may include the material information IF1 itself, which is described below. This information may be entered by the user or automatically by the user terminal 4.

[Activity A2.]

Next, the process proceeds to Activity A2, where the acquisition unit 231 acquires the substance information IF1. The acquisition unit 231, for example, acquires substance information IF1 transmitted from the user terminal 4. The acquisition unit 231 may acquire substance information IF1 from any database, such as a chemical substance database or crystal structure database, based on the transmitted information about the substance. The acquisition unit 231 may acquire each of the partial information comprising the substance information IF1 from separate sources. In other words, the information contained in substance information IF1 is not limited to those stored as a single data file.

<Substance Information IF1>

The substance information IF1 is information about a substance that contains at least one nucleus and at least one electron. The substance information IF1 includes a plurality of configuration candidates of the nuclei and the interaction V acting on each of the nucleus and the electron. The substance information IF1 may include the respective charge Z of a plurality of nuclei. In the present embodiment, the substance information IF1 contains information about the reference positions $R\_v0$ of the nuclei. Then, the acquisition unit 231 acquires configuration candidates of the nuclei, which is generated based on the reference positions $R\_v0$ of the nuclei by the candidate generation process described below.

The substance in this form is composed of at least one molecular system. Specifically, substance is composed of a molecular system, consisting of $n\_nucl$ nuclei and $n\_e$ electrons. Each particle has three spatial degrees of freedom in the x, y, and z directions. In the following description, electrons are treated quantum mechanically and nuclei are treated as classical point charges. That is, a configuration candidate of nuclei is described as a set of classical point charges. Nuclei shall be fixed in space. In addition, nuclei and electrons are sometimes referred to collectively simply as particles. Nuclei are not limited to those contained in neutral atoms but can also include nuclei contained in ions.

The processor 23 generates a Hamiltonian H corresponding to the energy of the material from such an interaction V. The Hamiltonian H may further include the kinetic energy T of the particles or the external potential $V\_ext$ for the particles.

The interaction V includes at least the electron-nucleus interaction $V\_en$. In this embodiment, the interaction V further includes the electron-electron interaction $V\_ee$ and the nucleus-nucleus interaction $V\_nn$. The interactions $V\_en$, $V\_nn$, and $V\_ee$ in this form are isotropic, which is determined by the distance between particles, but may be further anisotropic, which is determined by the orientation of the particles.

For the sake of explanation, let $Z\_v$ and $R\_v$ denote the charge and position of the vth nucleus, respectively, and assume each pair of these (n_e + n_nucl) particles (electron-electron, electron-nucleus, nucleus-nucleus) is subject to the common interaction V(d). d is the distance between the paired particles. For example, V(d) = 1/d for Coulomb interaction. Also, each electron is assumed to be subject to an external potential V_ext. The external potential V_ext is given by, for example, an electric field, magnetic field, heat, or disturbance.

The Hamiltonian H in the present embodiment is expressed as in number 1, with the position of the nucleus, R\_v, as a parameter. m_e is the electron mass. The r_1 with the hat symbol is the lth electron position operator. In the following description, the hat symbols may be omitted in the component numbers.

[number 1]

$$\mathcal{H}\left(\{\boldsymbol{R}_\nu\}_\nu\right) = \underbrace{\sum_{\ell=0}^{n_e-1} -\frac{1}{2m_e}\nabla_\ell^2}_{\equiv \hat{T}} + \underbrace{\frac{1}{2}\sum_{\ell=0}^{n_e-1}\sum_{\ell'=0}^{n_e-1} v\left(|\hat{\boldsymbol{r}}_\ell - \hat{\boldsymbol{r}}_{\ell'}|\right)}_{\equiv \hat{V}_{ee}} + \underbrace{\sum_{\ell=0}^{n_e-1}\sum_{\nu=0}^{n_{\mathrm{nucl}}-1} -Z_\nu v\left(|\hat{\boldsymbol{r}}_\ell - \boldsymbol{R}_\nu|\right)}_{\equiv \hat{V}_{en}}$$

$$+ \underbrace{\frac{1}{2}\sum_{\nu=0}^{n_{\mathrm{nucl}}-1}\sum_{\nu'=0}^{n_{\mathrm{nucl}}-1} Z_\nu Z_{\nu'} v\left(|\boldsymbol{R}_\nu - \boldsymbol{R}_{\nu'}|\right)}_{\equiv E_{\mathrm{nn}}} + \underbrace{\sum_{\ell=0}^{n_e-1} v_{\mathrm{ext}}\left(\hat{\boldsymbol{r}}_\ell\right)}_{\equiv \hat{V}_{\mathrm{ext}}}$$

Since the nuclei are treated as classical point charges in this formulation, the interaction V_nn between the nuclei in the Hamiltonian H can be a scalar quantity E_nn. This reduces the computational load on processor 23.

[Activity A3]

Next, the process proceeds to Activity A3, where the candidate generator unit 232 performs the acquired candidate generation process. In this way, the candidate generator unit 232 generates a plurality of configuration candidates of the nuclei. For example, the candidate generator unit 232 generates multiple configuration candidates of the nuclei by adding predetermined displacement ΔR to the obtained reference position R\_v0. For more details, the candidate generator unit 232 generates multiple configuration candidates of the nuclei by combining the reference position R\_v0 with the displacement ΔR\_v (v = 0, 1,..., n_nucl-1) from the reference position R \_v0 of the vth nucleus. The acquisition unit 231 then acquires the generated configuration candidates of the nuclei.

As an example, the candidate generator unit 232 first sets the maximum value ΔR\_vμmax of the displacement of the vth nucleus v in the μ direction (μ = x, y, z). For the sake of explanation, we will only discuss the case μ = x, but the same holds true for μ = y and z.

Next, the candidate generator unit 232 sets the position operator R\_{vx} of the nucleus for the state |j\_vx> \_n\_{qn} of the first qubits 321n, as in number 2. j\_vx = 0, 1,..., N\_{qn-1}, where N\_qn ≡ 2^{n\_qn} corresponds to the number of the generated configuration candidates of the nuclei for one degree of freedom.

[number 2]

$$\hat{R}_{\nu x}|j_{\nu x}\rangle_{n_{\mathrm{qn}}} \equiv \left(R_{\nu 0x} + j_{\nu x}\frac{\Delta R_{\nu x\mathrm{max}}}{N_{\mathrm{qn}}}\right)|j_{\nu x}\rangle_{n_{\mathrm{qn}}}\quad (j_{\nu x} = 0, 1, \ldots, N_{\mathrm{qn}} - 1)$$

This allows the position operator of the nucleus to represent discrete displacements from the reference position R\_v0. n_qn is the number of the first qubits 321n used to generate configuration candidates of the nuclei for one degree of freedom. The n_qn first qubits 321n constitute a quantum state |j\_vx> \_n_qn represented by the Kronecker product of 2^{n_qn} independent qubit states. Each of these independent quantum states is also called a computational basis. These computational bases are the eigenstates of the position operator R\_vx, as in number 2. n_qn is a parameter that gives the resolution of the search and does not necessarily have a direct relationship to the real size of the target molecule. Since the spatial degrees of freedom of the molecular system in the present embodiment is 3, 3 x n_qn first qubits 321n are used to represent configuration candidates of the nuclei in the molecular system. Therefore, the number of bases calculated to represent the position of one nucleus is 2^{3n_qn}.

For each of these computational bases, at least one of each of the configuration candidates of the nuclei obtained is assigned by the process described below. Therefore, each of these computational basis states $|j_v\rangle$ _3n_qn corresponds to the first qubit state. Hereinafter, for convenience of explanation, the first qubit state corresponding to the Jth configuration candidate of the nuclei may be denoted as $|J\rangle$ _{3n_{nucl}}_{n_qn} or simply $|J\rangle$.

The Hamiltonian H, shown in number 1, contains as a parameter a set of position coordinates of nuclei $\{R_v\}$. Therefore, in the present embodiment, the processor 23 can generalize the Hamiltonian H shown in number 1 to the Hamiltonian H~ as shown in number 3 by replacing the parameter with the position operator of the nucleus.

$$[\text{number } 3]$$

$$\widetilde{\mathcal{H}} \equiv \mathcal{H}(\{\hat{\boldsymbol{R}}_\nu\}_\nu)$$

The above generalization also defines V_en~, E_nn~, and V_ext~ by replacing the positions with the position operators in the nucleus-electron interaction V_en, the nuclear interaction energy E_nn, and the external potential V_ext in the original Hamiltonian H, respectively. For convenience of explanation, the tilde symbols (~) in V_en~, E_nn~, and V_ext~ may be hereinafter omitted.

[Activity A4]

Next, the process proceeds to activity A4, where the assignment unit 233 performs the assignment process. In this way, the assignment unit 233 assigns each of the acquired configuration candidates of the nuclei to at least one of the first qubit states $| J\rangle$ (i.e. computational basis). The first qubits state is composed of at least one first qubit 321n and is observable by observation operations on the first qubits. In the present embodiment, the assignment unit 233 assigns each of the configuration candidates of the nuclei described as classical point charges to at least one of the first qubit states. In the present embodiment, the assignment unit 233 assigns a configuration candidate of one nucleus to each of a plurality of computational bases. The first qubits 321n serve as computational qubits 321 to represent the configurations of the nuclei. The processing of activity A4 corresponds to the process that includes the assignment step in the present embodiment.

[Activity A5]

Next, the process proceeds to activity A5, where processor 23 performs a quantum circuit generation process based at least on the Hamiltonian H described above to generate a computational quantum circuit QC1 that specifies a series of quantum operations to be performed on the qubits 320. This computational quantum circuit QC1 of the present embodiment is generated as a command to the quantum computer 3 to perform quantum operations.

<Computational Quantum Circuit QC1>

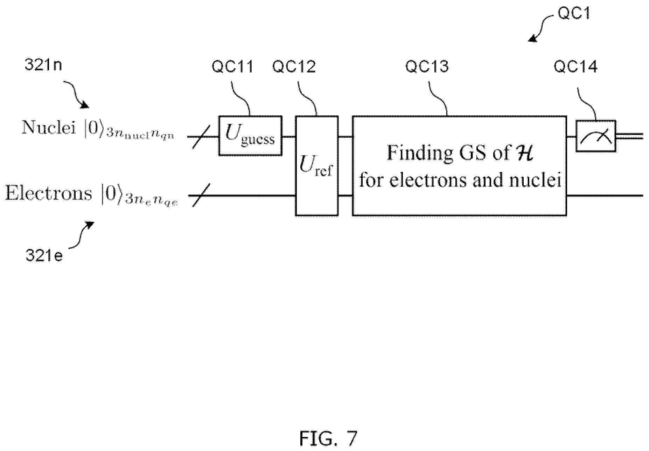

Here is an example of a computational quantum circuit QC1. FIG. 7 shows an example of a computational quantum circuit QC1.

The computational quantum circuit QC1 specifies a series of quantum operations to optimize the structure of the target system (in other words, material). Specifically, it consists of gate operations and measurements on the first qubits 321n representing the nuclear configurations and the second qubits 321e representing the multi-electron wavefunction. Specifically, the computational quantum circuit QC1 includes a first quantum gate operation QC11, a reference quantum gate operation QC12, a second quantum gate operation QC13, and an observation operation QC14. In detail, the computational qubit circuit QC1 is configured to act on at least the first qubit 321n and the second qubit 321e in order the first quantum gate operation QC11, the reference quantum gate operation QC12 and the second quantum gate operation QC13, and then perform the observation operation QC 14 on the first qubits 321n. In FIG.7 through FIG.16, the first quantum gate operation QC11 is also denoted as U_guess and the reference quantum gate operation QC12 is also denoted as reference circuit U_ref.

<First quantum gate operation QC11>

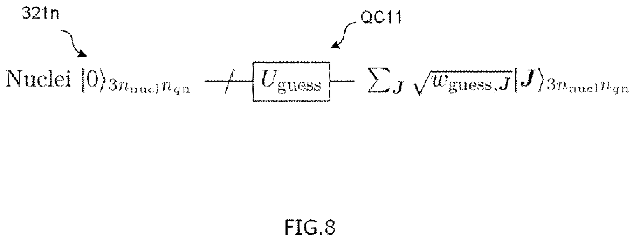

FIG. 8 illustrates an example of the first quantum gate operation QC11. The first quantum gate operation QC11 acts on the first qubits 321n to generate a superposition of multiple first qubit states. In the present embodiment, the first quantum gate operation QC11 acts on the initialized first qubits 321n to produce a superposition of first qubit states $|J\rangle$ with appropriate estimated weights w_{guess, J} assigned to each candidate for the optimal nuclear configuration. The process of generating the first quantum gate operation QC11 corresponds to the superposition step in the present embodiment. The estimated weight w_{guess,J} may be set equal for all J as an equal weight ratio or may be set to different values for each J based on other simulation results. The process of generating the first quantum gate operation QC11 is an example of a superposition step, i.e., the process of generating a superposition state of multiple first qubit states |J> by performing the prescribed first quantum gate operation QC11 on the first qubit 321n. The process of having the quantum computer 3 perform quantum operations based on the first quantum gate operation QC11 is also an example of a superposition step.

<Reference quantum gate operation QC12>

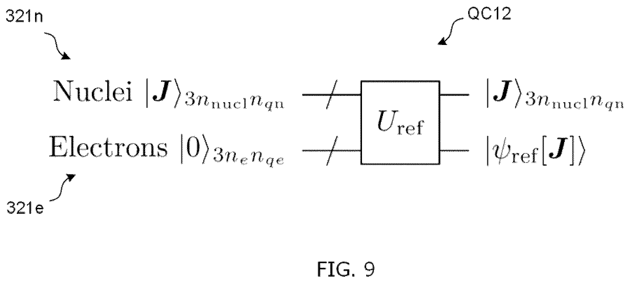

FIG. 9 illustrates an example of the reference quantum gate operation QC12. The reference quantum gate operation QC12 is performed on the qubits 320 containing the generated superposition states. A superposition state of reference electronic states is generated for each configuration candidate of the nuclei included in that superposition state. In the present embodiment, the reference quantum gate operation QC12 is performed on the superposition state of the plurality of first qubit states |J> generated by the first quantum gate operation QC11 above and the quantum state of the second qubit 321e representing the electronic state of the material. This generates the appropriate reference electronic state associated with each of the configuration candidates of the nuclei through the reference quantum gate operation QC12. The process of generating the reference quantum gate operation QC12 is an example of the electron placement step in this form. The process of having the quantum computer 3 perform quantum operations based on the reference quantum gate operation QC12 is also an example of an electron placement step.

<Second quantum gate operation QC13>

The second quantum gate operation QC13 includes the interaction V obtained for the first qubit representing the superposition state and the second qubit 321e. In detail, the second quantum gate operation QC13 corresponds to the Hamiltonian H (in the present embodiment, the generalized Hamiltonian H~) containing the acquired interaction V. In the present embodiment, the second quantum gate operation QC13 is performed on the qubits containing the superposition state of the reference electronic state generated through the reference quantum gate operation QC12. In detail, the second quantum gate operation QC13 produces a second qubit state representing an electronic state that can converge to the ground state in the configuration of the nuclei represented by the superposition state. The second quantum gate operation QC13 is also configured to search for the combination of nuclear displacements $\Delta R_\nu$ (opt) with the lowest ground state energy based on the generalized Hamiltonian H shown in number 3. This generates a correlated qubit state in which the first qubits 321n and the second qubits 321e are correlated. This process of generating the second quantum gate operation QC13 is an example of an interaction step. The process of having the quantum computer 3 perform quantum operations based on the second quantum gate operation QC13 is also an example of an interaction step.

The second quantum gate operation QC13 is configured to reduce the energy of the material in the quantum state represented by the first qubit 321n and second qubit 321e. For example, the second quantum gate operation QC13 is configured to perform a given ground state calculation method based on the first quantization form. The specific form of the ground state calculation method is arbitrary, such as the variational quantum eigensolver method (VQE) or the qubitization method. The probabilistic imaginary-time evolution method is employed as the ground-state calculation method in the present embodiment. The process such that the energy is lowered by the second quantum gate operation QC13 can also be called an energy minimization process. The energy minimization process includes at least one quantum gate operation. Since the probabilistic imaginary-time evolution method is employed in the present embodiment, the energy minimization process includes the observation operations of the ancillary bits 322. How to implement such ground-state calculation methods as quantum circuits is described below.

<Observation operation QC14>

As shown in FIG. 7, observation operation QC14 is an operation to observe a plurality of first qubits 321n. This projects the first qubits 321n to one of the states of the first qubits (i.e., computational bases) to which the configuration candidates of the nuclei are assigned. The observation operation QC14 is also known as a projection operation. The probability of observing a particular first qubit state increases with the lower energy of the ground state corresponding to the Hamiltonian H~. Therefore, among the superposition of multiple first qubit states in the correlated qubit state, the first qubit state with the lowest ground state energy is most likely to be observed. In other words, it is suggested that the configuration of the nuclei assigned to the first qubit state, which is observed in with a relatively high probability via the correlated qubit state, is the configuration with relatively low ground-state energy among the multiple configuration candidates of the nuclei. Through these observation operations, a ground state search is conducted.

Here is some theoretical background on the above ground state search.

As mentioned above, n_qe second qubits 321e are assigned in each direction for each electron to represent the multi-electron wave function in real space. In this case, the normalized multi-qubit state |Ψ> subject to optimization consists of 3 (n_{e}n_{qe} + n_{nucl}n_{qn}) computational qubits 321. The above multi-qubit state |Ψ> can be written as in number 4 by the Kronecker products of the first qubit state |J> and the second qubit state |K> using the coefficients c_K, J.

[number 4]

$$|\Psi\rangle = \sum_{\boldsymbol{K},\boldsymbol{J}} c_{\boldsymbol{K},\boldsymbol{J}}|\boldsymbol{K}\rangle_{3n_e n_{qe}} \otimes |\boldsymbol{J}\rangle_{3n_{\mathrm{nucl}} n_{qn}}$$

K is the collective notation of $3n\_e$ integers (each value 0 or greater and less than $2^{\wedge}n\_qe$) that specify the position eigenstates of a many-electron system. Also, J is the collective notation of $3n\_nucl$ integers (each value is 0 or greater and less than $2^{\wedge}n\_qn$).

The normalization condition is given by number 5.

[number 5]

$$\sum_{\boldsymbol{K},\boldsymbol{J}} |c_{\boldsymbol{K},\boldsymbol{J}}|^2 = 1$$

The weight w_J of the state of the Jth configuration of the nuclei is given by the number 6. For convenience of explanation, the Jth configuration of the nuclei is hereafter referred to simply as nuclear configuration J.

[number 6]

$$w_{\boldsymbol{J}} \equiv \sum_{\boldsymbol{K'}} |c_{\boldsymbol{K'},\boldsymbol{J}}|^2$$

The expansion factor c_K [J] is defined by number 7.

[number 7]

$$c_{\boldsymbol{K}}[\boldsymbol{J}] \equiv \frac{c_{\boldsymbol{K},\boldsymbol{J}}}{\sqrt{w_{\boldsymbol{J}}}}$$

The normalized multi-electron state $|\psi[J]\rangle$ associated with the nuclear configuration specified by the fixed J can be written as in number 8.

[number 8]

$$|\psi[\boldsymbol{J}]\rangle = \sum_{\boldsymbol{K}} c_{\boldsymbol{K}}[\boldsymbol{J}]|\boldsymbol{K}\rangle_{3n_e n_{qe}}$$

Number 4 can be written as in number 9 using number 8.

[number 9]

$$|\Psi\rangle = \sum_{\boldsymbol{J}} \sqrt{w_{\boldsymbol{J}}}|\psi[\boldsymbol{J}]\rangle \otimes |\boldsymbol{J}\rangle_{3n_{\mathrm{nucl}} n_{qn}}$$

For the multi-qubit state $|\Psi\rangle$ in number 9, the probability distribution of the nuclear configuration J obtained by measuring $3n\_\{nucl\}n\_\{qn\}$ qubits for nuclear displacement (i.e., the first qubits 321n) is the weight w_J itself of the nuclear configuration J. Therefore, when a ground state search with a sufficient number of steps (probabilistic imaginary time evolution or VQE in the present embodiment) is performed, the J(opt) that gives the maximum value of the distribution of w_J in the resulting multi-qubit state $|\Psi\rangle$ corresponds to the optimal nuclear configuration $\Delta R\_\nu\mu$ ($\nu = 0, 1,..., n\_\{nucl-1\}$, $\mu = x, y, z$).

In this way, the processor 23 generates a computational quantum circuit QC1 to identify the optimal nuclear configuration based on the information acquired.

[Activity A6.]

As shown in FIG. 6, the process then proceeds to activity A6, where processor 23 sends the generated computational quantum circuit QC1 to quantum computer 3. This allows the quantum processor 33 to acquire the computational quantum circuit QC1.

[Activity A7.]

Next, the process proceeds to activity A7, where the quantum processor 33 performs a quantum operation on the qubit 320 based on the acquired computational quantum circuit QC1. The quantum processor 33 thereby generates a correlated qubit state from the initialized first qubits 321n and second qubits 321e and observes the first qubits 321n in the correlated qubit state. The quantum processor 33 then sends the observation results (i.e., which first qubit state was observed) to the information processor apparatus 2.

[Activity A8.]

Next, at activity A8, the acquisition unit 231 acquires the observation results transmitted by the quantum processor 33.
The information processing system 1 repeats the process of activity A7 and activity A8 until the predetermined termination conditions are met. This allows the results of calculations on the quantum computer 3 to be stored in the information processor apparatus 2. The termination condition can be set arbitrarily, such as whether or not the number of repetitive steps reaches a predetermined value, or whether or not a termination operation is performed by the user. Based on the "observation results transmitted by the quantum processor 33," the acquisition unit 231 constructs a computational quantum circuit QC1 with the updated parameters and transmits it to the quantum processor 33. This allows for more accurate calculations to be performed.

[Activity A9.]

On the other hand, if the termination condition is met, the process proceeds to activity A9, where the processor 23 aggregates the observation results obtained by processing each activity A8. Here, the probability of observing each of the first qubit states depends on the weights $w\_J$ of the nuclear configuration J described above. Therefore, the aggregated observation results correspond to the weights $w\_J$ of the nuclear configuration J.

[Activity A10.]

Next, the process proceeds to activity A10, where the identification unit 235 identifies a configuration with relatively low energy among the configuration candidates of the nuclei based on the observation results for the first qubit 321n in the generated correlated qubit state. In detail, the identification unit 235 identifies, based on the observation results, the configuration of the nuclei that has the lowest energy eigenvalue of the ground state among the configuration candidates of the nuclei. For example, the identification unit 235 identifies the configuration candidate of the nuclei assigned to the first qubit state with the highest observed frequency as the configuration with relatively low energy among the configurations of the nuclei (in detail, the configuration of the nuclei with the lowest energy eigenvalue of the ground state). The processing of activity A10 is an example of the specification step in the present embodiment. The process in which the processor 23 causes the quantum computer 3 to perform the identification and obtains the result of the identification is also an example of the specification step.

[Activity A11.]

The process then proceeds to activity A11, where the processor 43 displays the configuration of the nuclei on the display unit 44. At this time, the processor 43 may display any information, such as the conditions of the calculation by the quantum computer 3 and the results of the calculation.

3. 2. An example of the second quantum gate operation QC13

In this chapter, the details of the second quantum gate operation QC13, which is generated in the information processing method described in the previous chapter, are explained using probabilistic imaginary-time evolution as an example. The second quantum gate operation QC13 described in this chapter is only an example.

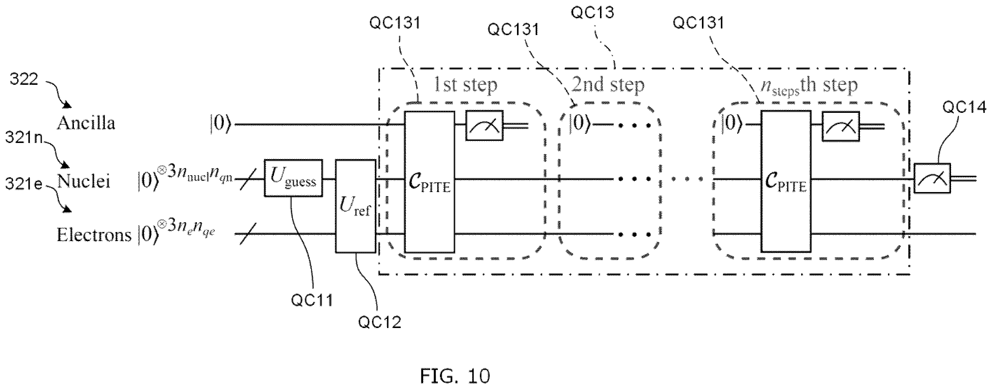

FIG. 10 illustrates the computational quantum circuit QC1 based on the probabilistic imaginary-time evolution method. The first quantum gate operation QC11 and the reference quantum gate operation QC12 are similar to those described in the previous chapter. In addition, in the present embodiment, the ancillary bits 322 are further used to implement the probabilistic imaginary-time evolution method on a quantum circuit. In the present embodiment, one ancillary bit 322 is denoted as "Ancilla", but the number of ancillary bits 322 may be two or more.

The second quantum gate operation QC13 includes at least one unit quantum circuit QC131. In detail, the second quantum gate operation QC13 is repeated a predetermined number of times (e.g., n_{steps} times) and is configured so that unit quantum circuit QC131 acts on the computed qubits 321 and ancillary bits 322.

<unit quantum circuit QC131>

The unit quantum circuit QC131 includes the search quantum circuit C_PITE and the observation operation on the ancillary bit 322. In detail, the unit quantum circuit QC131 is configured to perform the observation operation on the ancillary bits 322 after acting the search quantum circuit C_PITE on the first qubits 321n and the second qubits 321e.

<Search quantum circuit C_PITE>

The search quantum circuit C_PITE is a circuit that advances the imaginary time evolution by one step based on the Hamiltonian H~ of the target system defined by number 3. In this chapter, for convenience of explanation, Hamiltonian H~ will simply be referred to as Hamiltonian H. The search quantum circuit C_PITE contains a real-time evolution operator exp(-iHΔt) with Hamiltonian H as the generator. This allows the search quantum circuit C_PITE to be configured to perform an imaginary-time evolution method based on the first quantization form. Δt is the step width during real-time evolution.

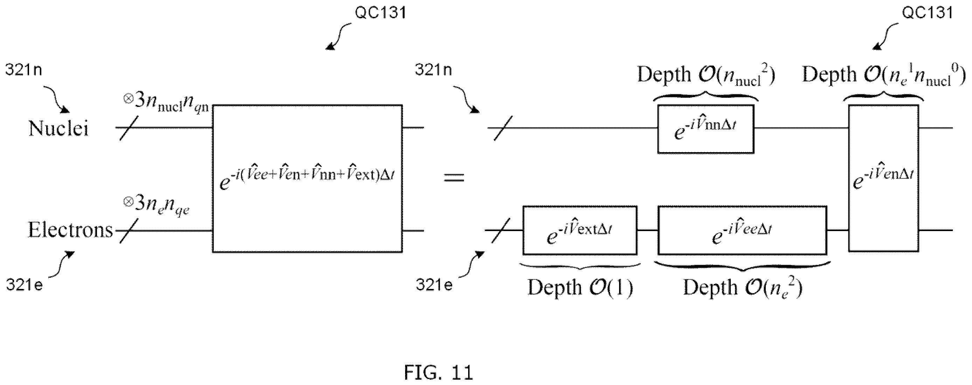

FIG. 11 shows an example of the expression of the contribution exp(-iVΔt) of the interaction V within the unit quantum circuit QC131. As shown in FIG. 11, the interaction part exp(-iVΔt) of the real-time evolution operator exp(-iHΔt) is denoted as exp(-i(V_ee + V_en + V_nn)Δt). The nuclear interaction V_nn acts only on the first qubit 321n, and the electron interaction V_ee acts only on the second qubit 321e. Therefore, these interacting parts are implemented as quantum gate operations acting only on the first qubit 321 n and those that acts only on the second qubit 321e, as shown in the right circuit of FIG. 6.

The number of combinations of two of the electrons in the target molecule is proportional to n_e^2, so the depth of circuit implementation of exp(-iV_eeΔt) is O(n_e^2). In the same way, we can also see that the depth of circuit implementation of exp(-iV_nnΔt) is O(n_nucl^2). n_nucl<<n_e for almost all molecules subject to practical calculations. The circuit implementation of exp(-iV_eeΔt) is independent of the presence or absence of nuclei. In light of these circumstances, the exp(-iV_enΔt) portion of exp(-iVΔt) does not affect the overall depth of the search quantum circuit C_PITE. Therefore, the contribution to the depth of the circuit implementing exp(-i(V_ee+V_en+V_nn)Δt) is mainly from exp(-iV_eeΔt).

Next, the gates exp(-iV_eeΔt), exp(-iV_nnΔt), exp(-iV_enΔt) and exp(-iV_extΔt) in the above search quantum circuit C_PITE The following is a description of the input-output correspondence for each of these gates. The method of implementation of each gate is arbitrary as long as the following correspondence is followed. Each gate can be implemented in circuit, for example, by expressing the function form of the interaction V as a polynomial and then implementing the polynomial by phase gates with existing techniques such as that described in the following non-patent document 1: P. J. Ollitrault, G. Mazzola, and I. Tavernelli, "Nonadiabatic molecular quantum dynamics with quantum computers. ", Phys. Rev. Lett. 125, 260511 (2020). or non-patent document 2: G. Benenti and G. Strini, "Quantum simulation of the single-particle Schroedinger equation.", American Journal of Physics 76, 657 (2008).

The position eigenvalue of a single electron is denoted as r^(k_x, k_y, k_z) using three integers k_x, k_y, k_z (each between 0 and 2^n_qe-1). The eigenstates corresponding to the position eigenvalues are denoted as | k_x, k_y, k_z>. The phase gate U_ee^(pair) acting on an electron pair is defined as in number 10 using the second qubit 321e.

[number 10]

$$U_{ee}^{(\text{pair})}(\Delta t) \overbrace{|\boldsymbol{k}\rangle_{3n_{qe}} \otimes |\boldsymbol{k'}\rangle_{3n_{qe}}}^{\text{A pair of electrons}}$$
$$\equiv \exp\left(-iv\left(\left|\boldsymbol{r}^{(\boldsymbol{k})} - \boldsymbol{r}^{(\boldsymbol{k'})}\right|\right)\Delta t\right)|\boldsymbol{k}\rangle_{3n_{qe}} \otimes |\boldsymbol{k'}\rangle_{3n_{qe}}$$

The real-time evolution operator exp(-iV_eeΔt) derived from the electron-electron interaction V_ee can be written as in number 11 by using the phase gate U_ee^(pair) . This means that exp(-iV_eeΔt) can be implemented with n_e(n_e-1)/2 U_ee^(pair) gates in a circuit of depth O(n_e^2).

$$[\text{number } 11]$$

$$e^{-i\hat{V}_{ee}\Delta t} = \prod_{\ell > \ell'} \exp\left(-iv\left(|\hat{\boldsymbol{r}}_\ell - \hat{\boldsymbol{r}}_{\ell'}|\right)\Delta t\right)$$

$$= \prod_{\ell > \ell'} \overbrace{U_{ee}^{(\text{pair})}(\Delta t)}^{\text{On } \ell\text{th and } \ell'\text{th electrons}}$$

As mentioned above, the displacement of the vth atom is represented as 3n_qn-qubit state | j_vx, j_vy, j_vz> _3n_qn. The phase gate U_nn^(v, v') acting on the 6n_q n-qubit state, which represents the displacement of the vth and v' th atom, is defined as in number 12.

$$[\text{number } 12]$$

$$U_{\text{nn}}^{(\nu,\nu')}(\Delta t) \quad \overbrace{|\boldsymbol{j}_\nu\rangle_{3n_{qn}}}^{\text{For } \nu\text{th nucleus}} \otimes \overbrace{|\boldsymbol{j}_{\nu'}\rangle_{3n_{qn}}}^{\text{For } \nu'\text{th nucleus}}$$

$$\equiv \exp\left(-iZ_\nu Z_{\nu'} v\left(|\boldsymbol{R}_\nu(\boldsymbol{j}_\nu) - \boldsymbol{R}_{\nu'}(\boldsymbol{j}_{\nu'})|\right)\Delta t\right)|\boldsymbol{j}_\nu\rangle_{3n_{qn}} \otimes |\boldsymbol{j}_{\nu'}\rangle_{3n_{qn}}$$

The real-time evolution operator exp(-iV_nnΔt), which originates from the nuclear interaction V_nn, can be written as in number 13 by using the phase gate U_nn^(v, v'). This means that exp(-iV_nnΔt) can be implemented in a circuit of depth O(n_nucl^2) from n_nucl(n_nucl-1)/2 U_nn^(nu, nu') gates.

$$[\text{number } 13]$$

$$e^{-i\hat{V}_{\text{nn}}\Delta t} = \prod_{\nu > \nu'} \exp\left(-iZ_\nu Z_{\nu'} v\left(|\hat{\boldsymbol{R}}_\nu - \hat{\boldsymbol{R}}_{\nu'}|\right)\Delta t\right)$$

$$= \prod_{\nu > \nu'} \overbrace{U_{\text{nn}}^{(\nu,\nu')}(\Delta t)}^{\text{On } \nu\text{th and } \nu'\text{th nuclei}}$$

The phase gate U_en^(v) acting on a single electron and the vth nucleus is defined as in number 14.

$$[\text{number } 14]$$

$$U_{\text{en}}^{(\nu)}(\Delta t) \quad \overbrace{|\boldsymbol{k}\rangle_{3n_{qe}}}^{\text{For an electron}} \otimes \overbrace{|\boldsymbol{j}_\nu\rangle_{3n_{qn}}}^{\text{For } \nu\text{th nucleus}}$$

$$\equiv \exp\left(iZ_\nu v\left(\left|\boldsymbol{r}^{(\boldsymbol{k})} - \boldsymbol{R}_\nu(\boldsymbol{j}_\nu)\right|\right)\Delta t\right)|\boldsymbol{k}\rangle_{3n_{qe}} \otimes |\boldsymbol{j}_\nu\rangle_{3n_{qn}}$$

The real-time evolution operator exp(-iV_enΔt) derived from the electron-nucleus interaction V_en can be written as in number 15 by using the phase gate U_en^(v).

[number 15 ]

$$e^{-i\hat{V}_{\mathrm{en}}\Delta t} = \prod_{\ell=0}^{n_e-1} \prod_{\nu=0}^{n_{\mathrm{nucl}}-1} \exp\left( iZ_\nu v\left( |\hat{\boldsymbol{r}}_\ell - \hat{\boldsymbol{R}}_\nu| \right) \Delta t \right)$$

$$= \prod_{\ell=0}^{n_e-1} \prod_{\nu=0}^{n_{\mathrm{nucl}}-1} \overbrace{U_{\mathrm{en}}^{(\nu)}(\Delta t)}^{\text{On } \ell\text{th electron and } \nu\text{th nucleus}}$$

The real-time evolution operator exp(-iV_enΔt) derived from the electron-nucleus interaction V_en can also be written as in number 16. This means that the real-time evolution operator exp(-iV_enΔt) can be implemented using n_e n_nucl U_en^(v) gates.

[Number 16 ]

$$e^{-i\hat{V}_{\mathrm{en}}\Delta t} = \prod_{d=0}^{n_e-1} \prod_{\nu=0}^{n_{\mathrm{nucl}}-1} \overbrace{U_{\mathrm{en}}^{(\nu)}(\Delta t)}^{\text{On } \ell\text{th electron and } \nu\text{th nucleus}} \Bigg|_{\ell=\nu+d \bmod n_e}$$

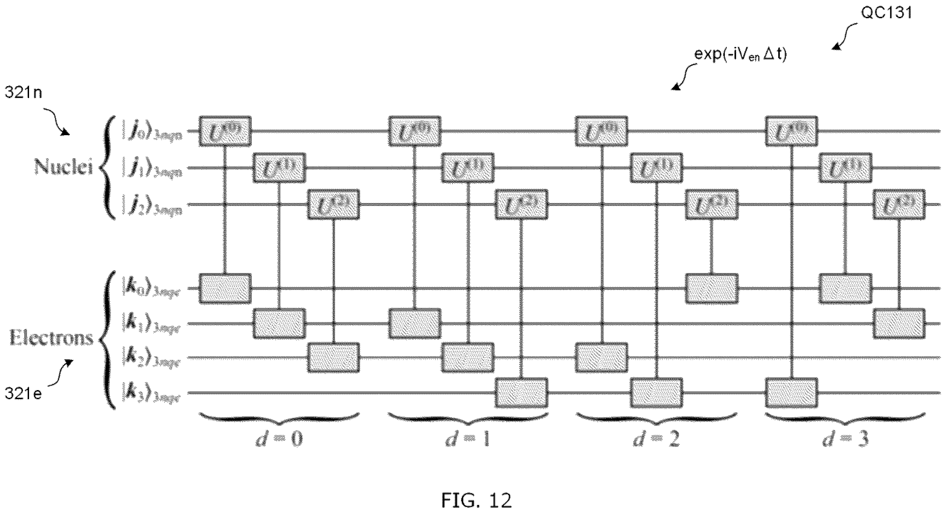

The real-time evolution operator exp(-iV_enΔt) can be implemented in a circuit of depth O(n_e^1 n_nucl^0) with respect to n_e and n_nucl by using number 16. FIG. 12 shows an example of a quantum circuit for implementing the real-time evolution operator exp(-iV_enΔt) in the case n_e=4 and n_nucl=3.

The phase gate U_ext acting on a single electron is defined as in number 17.

[number 17]

$$U_{\mathrm{ext}}(\Delta t) \quad \overbrace{|\boldsymbol{k}\rangle_{3n_{qe}}}^{\text{For an electron}} \equiv \exp\left( -iv_{\mathrm{ext}}(\boldsymbol{r}^{(k)})\Delta t \right) |\boldsymbol{k}\rangle_{3n_{qe}}$$

The real-time evolution operator exp(-iV_extΔt) derived from the external potential can be written as in number 18 using the phase gate U_ext.

[number 18]

$$e^{-i\hat{V}_{\mathrm{ext}}\Delta t} = \prod_{\ell=0}^{n_e-1} \exp\left( -iv_{\mathrm{ext}}(\hat{\boldsymbol{r}}_\ell)\Delta t \right)$$

$$= \prod_{\ell=0}^{n_e-1} \overbrace{U_{\mathrm{ext}}(\Delta t)}^{\text{On } \ell\text{th electron}}$$

This means that exp(-iV_extΔt) can be implemented in a circuit of depth O(1) with n_e U_ext gates.

The above explanation was a way to generate candidate structures by displacing all of the n_nucl nuclei. If the positions of some (e.g., n_nucl') specific nuclei among the n_nucl nuclei are subject to optimization and the positions of the other n_nucl-n_nucl' nuclei are not subject to optimization (e.g., the other nuclei positions are fixed), the potential due to the latter should be absorbed in the definition of the external potential v_ext. This allows the method described above to be applied to n_nucl' nuclei.

3.3 Example of a search quantum circuit C_PITE

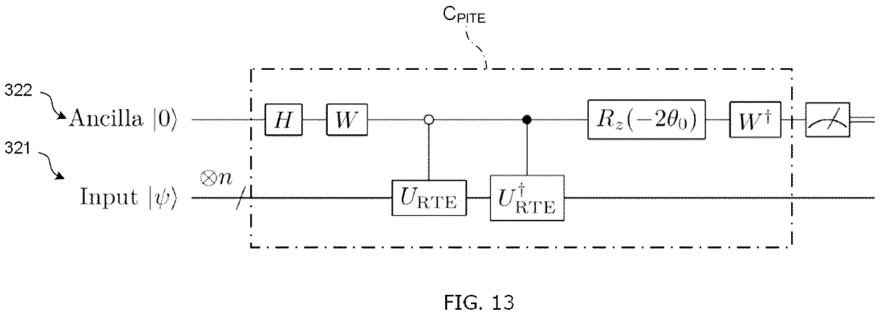

Here, we explain an example of the configuration of the search quantum circuit C_PITE in the case where the imaginary-time evolution method is adopted as the ground state calculation method. FIG. 13 shows an example of the search quantum circuit C_PITE for imaginary time evolution. In FIG. 13, the real-time evolution operator exp(-iH$\Delta$t) is denoted as U_{RTE}. The search quantum circuit C_PITE is configured to perform the Hadamard gate operation H and the W gate operation on ancillary bit 322 in sequence, then the control NOT gate operation with ancillary bit 322 as the control bit, then the rotational gate operation R_z(-2θ_0) and the Hermitian conjugate of the W gate The operation is configured to be performed on ancillary bit 322. The control NOT gate operation has the ancillary bit 322 set as the control bit and the computational qubit 321 set as the target bit. The control NOT gate operation is configured such that depending on the state of the ancillary bit 322, it determines whether the forward real-time evolution operator U_{RTE} or the backward real-time evolution operator U^†_{RTE} is acted on the computational qubit 321. The specific configuration of the search quantum circuit C_PITE is not limited to this and is arbitrary.

The search quantum circuit C_PITE is configured to act on the |ψ>*|0> state(* indicates the Kronecker product), which consists of the state |ψ> of the computational qubit 321 and the state |0> of the auxiliary bit 322. This gives a probabilistic state in which the imaginary time evolution operator exp(-H$\Delta\tau$), with the general Hermitian operator H as the generator, acts on such a multi-qubit system. Such a quantum circuit C_PITE is constructed to be polynomially expandable with time step $\Delta\tau$ as a parameter. Note that $\Delta\tau$ is the step width of the imaginary time evolution method. Polynomial expansions can be expansions up to the first order range of $\Delta\tau$ or expansions involving terms of second or higher order.

The real-time evolution operator exp(-iH$\Delta$t) can be approximated by the combination of the kinetic energy contribution exp(-iT$\Delta$t) and the interaction V contribution exp(-iV$\Delta$t). In this form, as shown in number 3, the Hamiltonian H contains the nuclear coordinate R_ν explicitly. Therefore, the contribution of the interaction V, exp(-iV$\Delta$t), must be implemented within the search quantum circuit C_PITE.

<Quantum Amplification Circuit Q>

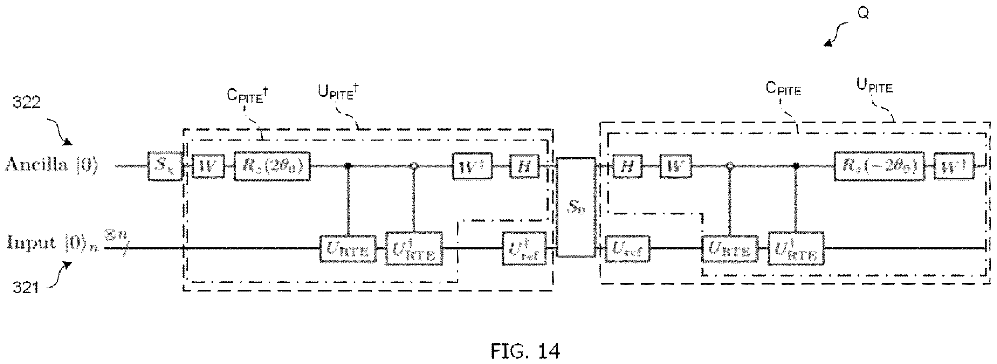

If the second quantum gating operation QC13 includes the observation operation of the auxiliary bit 322, the second quantum gating operation QC13 may further be equipped with a quantum amplification circuit Q. FIG. 14 shows an example of a quantum amplitude amplifier circuit Q in the imaginary-time evolution method. The quantum amplification circuit Q is a quantum circuit that can improve the probability that the auxiliary bit 322 is observed as a given state. The amplitude amplification operator Q in question acts on the oracle S_x and then the zero-reflection S_0 sandwiched between U_PITE and U_PITE^†. Specifically, the amplitude amplification operator Q is defined by the number 19.

[number 19]

$$Q \equiv -\mathcal{U}_{\mathrm{PITE}} S_0 \mathcal{U}_{\mathrm{PITE}}^{\dagger} S_{\chi}$$

The quantum circuit U_PITE is configured to act on a multi-qubit state that includes a 321 system of computational qubits and a 322 system of auxiliary bits. The quantum circuit U_PITE is configured to act in turn on the reference circuit U_ref, which acts on the relevant multi-qubit, and the quantum circuit C_PITE, which implements the real-time evolution operator exp(-iκΘ) with the Hermite operatorΘ as generator. In detail, the quantum circuit U_PITE is represented by the product of the reference circuit U_ref and the quantum circuit C_PITE as in number 15.

[number 20]
$$\mathcal{U}_{\mathrm{PITE}} \equiv \mathcal{C}_{\mathrm{PITE}}(U_{\mathrm{ref}} \otimes I_2)$$

<Zero reflection S_0>

The reflection operator S_φ is an operation that reverses the sign of a specific state |φ> among the N qubit states, but does not change the other states. A reflection operator S_φ whose specific state |φ> is |0...0> is called a zero reflection S_0.

<Oracle S_x>

The oracle S_x performs an operation that reverses the sign of the state orthogonal to the state to be amplified. Zero reflection S_0 can be implemented as an algorithm-independent quantum circuit combined with quantum amplitude amplification. The oracle S_x can be implemented as a quantum circuit for such an algorithm (the imaginary-time evolution method in this implementation) as appropriate.

3.4. Simulation Results for One-Electron Molecular Systems

In this section, we describe simulation results when the above information processing method is applied to a one-electron molecular system. In this simulation, the material subject to structural optimization is a molecular system consisting of a single electron and two nuclei. In this simulation, the above information processing was performed on a classical computer to simulate a quantum computation performed by the procedure in FIG. 6.

To simplify the problem setting, space is assumed to be one-dimensional. The physical quantity values that appear below are in atomic units. The electrons were treated as having charge -1 and the two nuclei as both having charge + 1. The position of one of the nuclei was fixed and the distance from it to the other was divided into 8 parts from 1.5 to 3. As the initial electronic state at each nuclear configuration, the electronic wave function with a larger amplitude at deeper electrostatic potentials due to the nuclei was used as the initial electronic state. Such an initial electronic state is represented as a second qubit state using the second qubit 321e.

Stochastic imaginary-time evolution was employed as the ground-state search method.

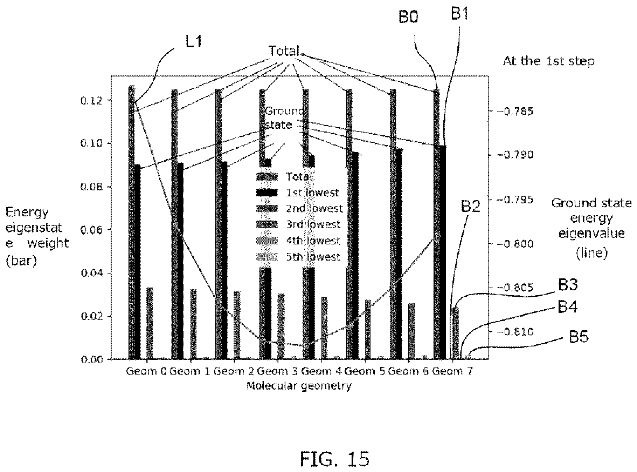

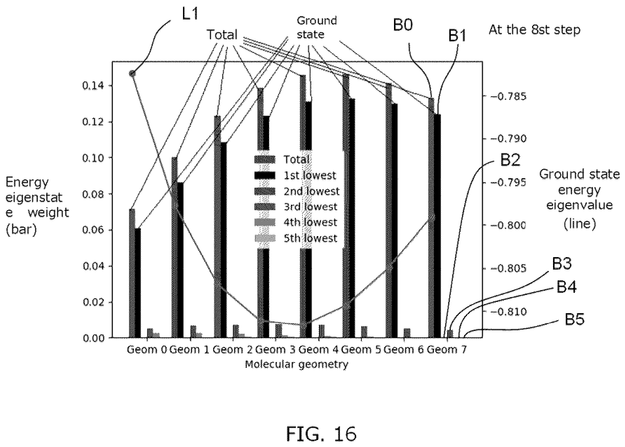

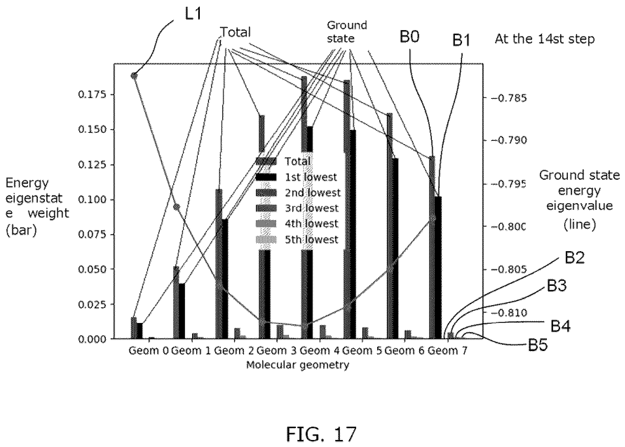

FIG. 15 shows the simulation results of the first step (i.e., the initial state). FIG. 16 shows the simulation results for the eighth step. FIG. 17 shows the simulation results of the 14th step.

The labels (Geom 0 to Geom 7) on the horizontal axis in FIG.15 to FIG.17 indicate candidate nuclei configurations.

Bars B1 to B5 in FIG.15 to FIG.17 show the weights of each energy eigenstate from the ground state (1st lowest) to the 4th excited state (5th lowest), respectively, and bars B0 show the sum (total) of the weights of these energy eigenstates, respectively.

The line graph L1 in FIG.15-FIG.17 shows the energy eigenvalues of the ground state. As shown in the line graph L1, the fifth nuclear configuration from the left (Geom 4) has the lowest total energy eigenvalue of the ground state and is the optimal nuclear configuration.

As shown in FIG. 15, the first quantum gate operation QC11 in this simulation gives equal weight to all candidate nuclear configurations in the initial state. Therefore, the weights of the energy eigenstates in the initial electron wave function were all equal.

As shown in FIG.16 and FIG.17, for each step in the iterative ground-state search, the weight of the optimal configuration (i.e., the configuration of nuclei with the lowest ground-state energy eigenvalues) was the largest, and as the deviation from the optimal configuration increased, the weight of candidate nuclear configurations decreased The weights of the candidate nuclei configurations decreased as the deviations from the optimal configuration increased.

From these results, the observation of the first qubit state after the ground-state search is performed maximizes the observation probability of the optimal configuration (i.e., the configuration of nuclei with the lowest ground-state energy eigenvalue) among the multiple candidate configurations of nuclei. This result suggests that the optimal molecular structure corresponding to these interatomic distances, i.e., Geom 4 with the lowest ground-state energy, can be obtained through the observation of the first qubit state by the above information processing. Therefore, it is shown that by employing this information processing method, it is possible to identify the most stable nuclear configuration for the Hamiltonian H containing the obtained interaction V.

3.5. Simulation Results for Multi-Electron Molecular Systems

In this section, we explain the simulation results when the above information processing method is applied to a molecular system consisting of two electrons and two nuclei. In this simulation, unlike the simulations in the previous section, electron-electron interactions are taken into account. In this simulation, the simulation of a quantum computation performed by the information processing procedure shown in FIG. 6 was also performed on a classical computer.

The position of one of the nuclei was fixed and the distance from it to the other was divided into 8 parts, each corresponding to a candidate structure (Geom0 to Geom7) with equal weight, and the step was proceeded.

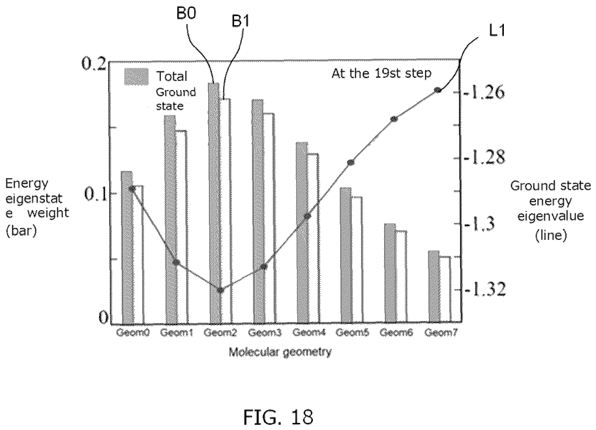

FIG. 18 shows the simulation results of the weights of each candidate structure immediately after 19 steps. The line graph L1 in FIG. 18 shows the energy eigenvalues of the ground state. As shown in the line graph L1, the third nuclear configuration from the left (Geom 2) has the lowest total energy eigenvalue of the ground state and is the optimal nuclear configuration. In FIG. 18, bar B1 shows the weights of the energy eigenstates in the ground state (1st lowest), and bar B0 shows the sum (total) of the weights of each energy eigenstate from the ground state (1st lowest) to the fourth excited state (5th lowest).

It can be seen that among several candidate configurations of nuclei, the observed probability of the optimal configuration (Geom 2 in the present case) is the largest. This shows that this information process gives correct results even in the presence of electron-electron interactions.

4. another example of information processing method

This chapter describes an example of information processing in the case where the computational quantum circuit QC1 can be updated based on predetermined parameters. Specifically, the variational eigenvalue solver method (hereinafter referred to as VQE) is used as the ground state calculation method. The following is another example of information processing when the variational eigensolver method (hereinafter referred to as VQE) is employed as the ground state calculation method. Explanations of information processing in this chapter that are common to those in the previous chapters may be omitted by appending the same part number. The information processing described in this chapter is sometimes referred to as secondary information processing to distinguish it from the primary information processing.

4.1.1. Information processing flow

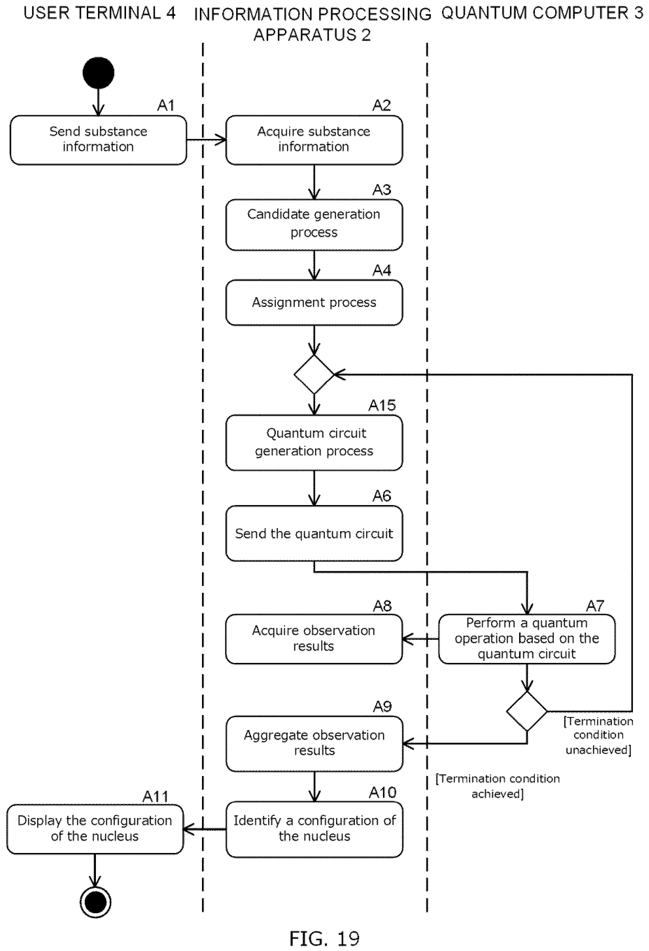

FIG. 19 is an activity diagram showing an overview of the information processing in this form performed in the information processing system 1. The information processing may include optional exception processing that is not illustrated in the activity diagram. Exception processing includes interruption of the relevant information processing or omission of each process. The selection or input of information in this information processing can be based on operations by the user or can be done automatically without the user's operation.

This form of information processing differs from the information processing in the previous chapter in that it can be performed based on the observation results in Activity A8 during the circuit generation process in Activity A15. Specifically, after processing Activity A1~ Activity A4, the information processing system 1 executes the quantum circuit generation process in Activity A15. At this time, processor 23 further generates a computational quantum circuit QC1 based on predetermined parameters. Specifically, processor 23 sets the quantum gate operations included in the computational quantum circuit QC1 according to certain parameters. Such parameters include, for example, the rotation angle (phase angle) of the rotary gate operation. For the first activity A15, processor 23 may generate the computational quantum circuit QC1 based on the initial values of the predetermined parameters. The details of the relevant computational quantum circuit QC1 and its parameters are described below.

Next, Activity A6~ Activity A8 is processed to obtain the observation results from the quantum computation based on the computational quantum circuit QC1 generated in Activity A15. Then, if the termination condition has not been achieved, the process returns to activity A15.

At this time, processor 23 calculates the parameters used to generate the computational quantum circuit QC1 at activity A15 based on the observations obtained at activity A8. This increases the likelihood that the observed state will lead to the ground state, as the computational quantum circuit QC1 is regenerated accordingly to reflect the observation results.

On the other hand, if the termination condition is achieved, Activity A9~ Activity A10 is processed to identify the optimal nuclear arrangement.

4.2. An example of a computational quantum circuit QC1 in VQE

This section describes an example of how to generate a computational quantum circuit QC1 when VQE is employed as the ground state calculation method used in the information processing in the previous section.

<Hypothetical state

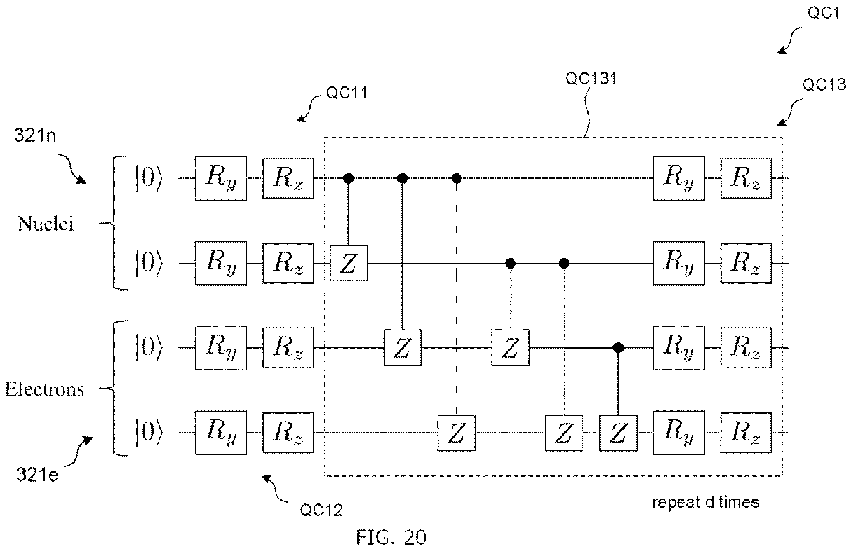

FIG. 20 is an example of a computational quantum circuit QC1 for VQE. In this section, the computational quantum circuit QC1 for VQE is simply referred to as the computational quantum circuit QC1. The computational quantum circuit QC1 consists of a rotational gate operation ($R_y$ and $R_z$ gate operations) and a two-qubit gate operation (control Z-gate) characterized by $N_p$ arbitrary real parameters $\theta = \{\theta_1, ..., \theta_{N_p}\}$. $N_p$ arbitrary real parameters $\theta = \{\theta_1, ..., \theta_{N_p}\}$ correspond to the prescribed parameters for generating the computational quantum circuit QC1 in the above information processing.

Specifically, the computational quantum circuit QC1 is configured to first perform a rotational gate operation on each of the first 321n qubits. This results in a superposition of the first qubit state composed by the first 321n qubits. Therefore, the rotary gate operation on the 321 computational qubits concerned includes the function as the first quantum gate operation QC11.

On the other hand, the computational quantum circuit QC1 is configured to perform a rotational gate operation on the second qubit 321e. The parameters of these rotational gate operations are explicitly or implicitly correlated with the first qubit state. Therefore, the rotational gate operation on said second qubit 321e includes the function as a reference quantum gate operation QC12.

The computational quantum circuit QC1 is then configured to perform the second quantum gate operation QC13.

The second quantum gate operation QC13 can be constructed by repeating the smallest unit (i.e., unit quantum circuit QC131) configured to perform a two-qubit gate operation followed by a rotary gate operation, for any number d. This form of two-qubit gate operation is a controlled Z-gate operation. The said controlled Z-gate operation can represent an interaction between each particle.

The computational quantum circuit QC1 for VQE shown in FIG. 20 is an example. The computational quantum circuit QC1 for VQE can be any quantum circuit constructed from parameterized gate operations. For example, a computational quantum circuit QC1 may be constructed based on physical considerations, such as unitary coupled cluster hypothetical states.

<Optimization Algorithm

In the VQE method, the acquisition unit 231 acquires the ground state by updating the parameters contained in the computational quantum circuit QC1 so as to minimize the energy (or cost function). Here, the process for updating parameters is performed by the information processor «apparatus/device» 2. In this chapter, the variational imaginary-time evolution method is used to update the parameters contained in the computational quantum circuit QC1. The variational imaginary time evolution method is based on the non-patent: S. McArdle, et al, "Variational ansatz-based quantum simulation of imaginary time evolution ", npj Quantum Information, 5, 75 (2019). In the variational imaginary-time evolution method, the parameters are given an imaginary-time dependence, $\theta(\tau)$, and the parameters are determined so that the time evolution in the imaginary-time direction is reproduced with each step. Solve the linear equation given by number 21 to determine the parameters in the next step.

$$[\text{number } 21]$$
$$M\dot{\boldsymbol{\theta}} = \boldsymbol{C}$$

where the matrices M and vectors C are given by numbers 22 and 23, respectively.

$$[\text{number } 22]$$
$$M_{pq} = \mathrm{Re}\left( \frac{\partial\langle\phi(\boldsymbol{\theta}(\tau))|}{\partial\theta_p} \frac{\partial|\phi(\boldsymbol{\theta}(\tau))\rangle}{\partial\theta_q} \right)$$

$$[\text{number } 23]$$
$$C_p = -\mathrm{Re}\left( \frac{\partial\langle\phi(\boldsymbol{\theta}(\tau))|}{\partial\theta_p} \hat{H}|\phi(\boldsymbol{\theta}(\tau))\rangle \right)$$

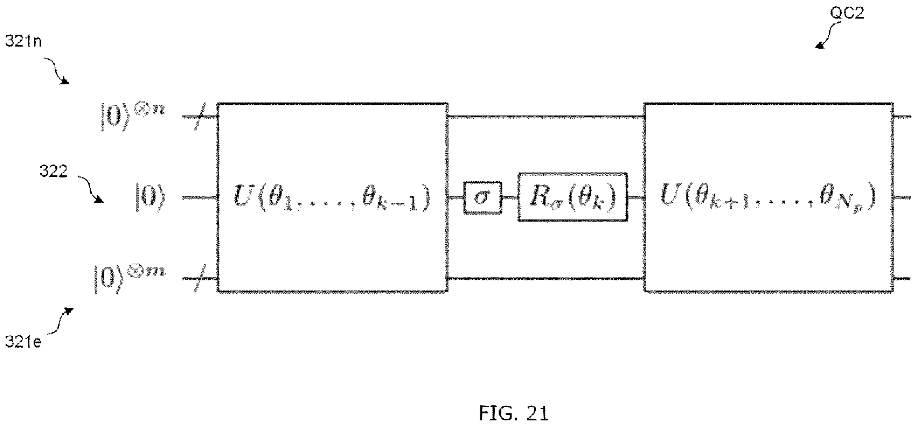

FIG. 21 shows the differential circuit QC2 for computing the derivative $(-2i)\text{-}\partial|\varphi(\theta)\rangle/\partial\theta\_k$ with respect to $\theta\_k$. The parameter derivative of the computational quantum circuit QC1 included in number 22 can be computed using the derivative circuit QC2. The unitary re operation that depends on $\theta\_1,...,\theta\_{k-1}$ is denoted $U(\theta\_1,...,\theta\_{k-1})$. The same is true for $U(\theta\_{k-1},..., \theta\_{N\_p})$. The $R\_\sigma$ gate ($\sigma$ = x, y, z) is a rotational gate operation.

The number 23 can be calculated from the sum of the results of dividing the Hamiltonian H into the kinetic term T and the potential term V, respectively. First, we discuss the potential term V. Since the potential term V can be expressed in terms of a diagonal matrix with respect to the computational basis, it can be calculated from the absolute squares of the coefficients obtained by repeatedly measuring each of the computational quantum circuit QC1 and differential circuit QC2 in the computational basis. We then discuss the kinetic term T. The kinetic term T can be represented by a diagonal matrix in momentum space. Therefore, processor 23 first transforms the wavefunction into momentum space by applying a quantum Fourier transform to the computational quantum circuit QC1 and differential circuit QC2. Processor 23 then iteratively measures the computational quantum circuit QC1 and differential circuit QC2, respectively, in the computational basis for the kinetic term T, as well as the potential term V. This allows the acquisition unit 231 to obtain the absolute squares of the coefficients. Processor 23 can calculate the number related to the kinetic term T from said absolute square.

Since the imaginary time derivative of the parameters can be obtained from the number 21, the parameters in the next step can be determined from the number 24 according to the Euler method, for example. where $\Delta\tau$ is the imaginary time step width.

[number 24]

$$\boldsymbol{\theta}(\tau + \Delta\tau) = \boldsymbol{\theta}(\tau) + \Delta\tau\dot{\boldsymbol{\theta}}$$

This chapter describes the variational imaginary-time evolution method as an optimization algorithm, but this is only an example. Other optimization algorithms may be used.

The description in this section regarding the method used to compute the ground state of the Hamiltonian H using a single computational quantum circuit QC1 is only an example. The ground state may be calculated by the information processor «apparatus/device» 2 as a post-processing step from the results of a calculation using multiple computational quantum circuits QC1.

4.3.3. Simulation based on VQE

In this section, we describe simulation results when VQE is employed as the ground state search method. The calculation conditions are the same as in the examples in FIGS. 15-17. Specifically, the initial state is the state generated by the computational quantum circuit QC1, which is characterized by parameters generated by random numbers.

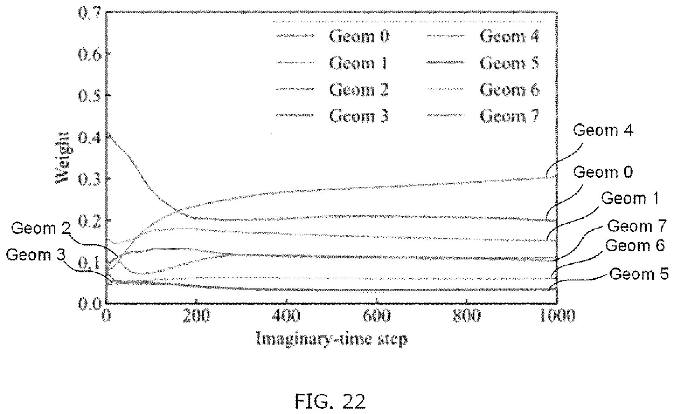

FIG. 22 shows the simulation results of the sum of the weights of the energy eigenstates for each nuclear configuration (Geom 0 to Geom 7). The horizontal axis of FIG. 22 corresponds to the number of steps of imaginary time evolution. The vertical axis of FIG. 22 corresponds to the sum of the weights of the energy eigenstates. As shown in FIG. 22, it can be seen that the weight of the optimal placement (Geom 4) is amplified as the imaginary time evolution step increases.

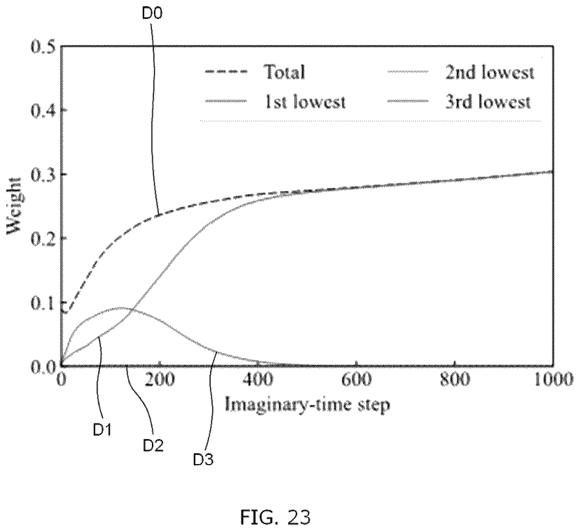

FIG. 23 shows the weights of the ground state (1st lowest), first excited state (2nd lowest), second excited state (3rd lowest), and sum (Total) energy eigenstates of the electronic wave function in the optimal configuration (Geom 4). The horizontal axis of FIG. 23 corresponds to the number of steps of imaginary time evolution. The vertical axis in FIG. 23 corresponds to the weights of the energy eigenstates. In FIG. 23, D0 shows the plot of the total sum (Total), D1 shows the plot of the ground state (1st lowest), D2 shows the plot of the first excited state (2nd lowest), and D3 shows the plot of the second excited state (3rd lowest). As shown in FIG. 23, it can be seen that with increasing steps of imaginary time evolution, the ratio of the weights of the ground state (1st lowest) to the sum of the weights increases relative to the ratio of the weights of the other states. This implies that the weights of the ground state are amplified by the computational quantum circuit QC1 (especially the second quantum gate operation QC13).

5. other

The above information processing system 1 may be further inventive in the following ways.

The substance does not have to be a single molecule, it can be a system of multiple molecules, a metal containing metal atoms or ions, a metal oxide, etc. The substance may have no periodic or quasi-periodic structure, such as a single molecule or amorphous, or it may have a periodic structure, such as polycrystalline or single crystal, or a combination of these. In other words, the substance should contain at least one nucleus and at least one electron. Furthermore, if interaction by electrons is not considered, the substance need only contain at least one nucleus.

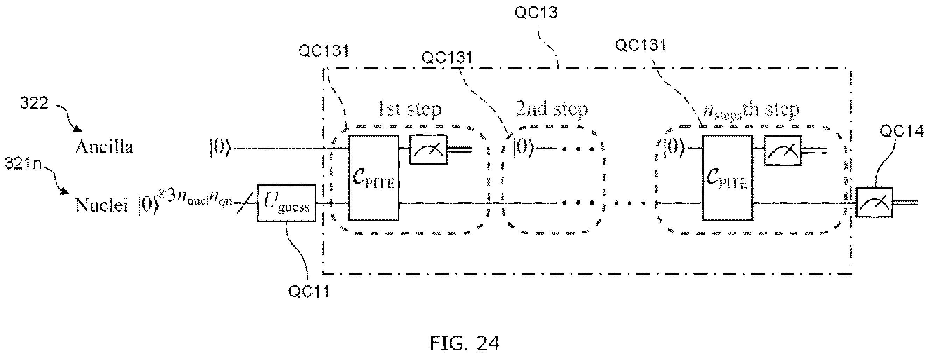

In detail, the above information processing method optimizes both the nuclear configuration and the multi-electron state in parallel, but it is also applicable to $3n\_nucln\_qn$ qubit systems that consider only the nuclear configuration. FIG. 24 shows an example of a unit quantum circuit QC131 for information processing methods for a qubit system that considers only the nuclear configuration. The second quantum gate operation QC13 shown in FIG. 24 is composed of the parts other than those related to the second qubit 321e in the second quantum gate operation QC13 shown in FIG. 5. In this case, among the time evolution operators based on the interaction V, only the time evolution operator $\exp(-iV\_nn\Delta t)$ based on the interatomic interaction $V\_nn$ needs to be implemented on the unit quantum circuit QC131. This allows the structural optimization of systems consisting only of classical point charges to be performed. In this case, the circuit depth is $O(n\_nucl^2)$.

Furthermore, by setting the interaction V appropriately, the particles are not limited to nuclei or electrons, but can employ any particle. For example, if the substance is composed of multiple molecules, the molecules should be treated as a single particle, and the above information processing method should be performed after setting inter-particle interactions Vpp (e.g., hydrogen bonds, Coulomb bonds, van der Waals bonds, etc.). In this case, the operator corresponding to the energy is not limited to the Hamiltonian, but can be any operator corresponding to the energy, such as the Gibbs energy, as appropriate for the system to focus on.

In other words, the substance information IF1 is information about a substance that contains at least one particle, and include multiple configuration candidates of the particles and interactions that act on the particles. In this case, the interaction V should include at least the particle-particle interaction Vpp, which acts between the particles. In this case, the acquisition unit 231 performs the same process of acquiring the substance information IF1 as in Activity A2 above. The assignment unit 233 assigns each of the acquired configuration candidates of the particles to at least one of the first qubit states, similar to activity A4 described above. The first qubit state is composed of at least one first qubit and is observable by an observation operation on the first qubit. The quantum manipulation unit 234 performs the prescribed first quantum gate operation QC11 on the first qubits 321n to generate a superposition state of multiple first qubit states. The quantum manipulation unit 234 performs the second quantum gate operation QC13 involving the acquired interaction V on the first qubits 321n representing the superposition state, thereby generating a correlated qubit state with correlation between the first qubits 321n. As a result, a computational quantum circuit QC1 is generated through the same process as Activity A5, and the computational quantum circuit QC1 is executed by the quantum processor 33. Then, similar to activity A10 described above, the identification unit 235 performs a process to identify the configuration with relatively low energy among the configuration candidates of the particles based on the observation results for the first qubit 321n with the generated correlated qubit state.

The number of particle types may be one type of nucleus only, two types of nucleus and electrons, or three or more types including other particles. In other words, the number of particle types is arbitrary. Therefore, the above information processing method can be applied to complex biological systems composed of multiple types of molecules.

In the above information processing method, nuclei are treated as classical point charges and their ground states are obtained without considering their kinetic energy at all, but the method is equally applicable when nuclei are quantum mechanical particles. For example, the assignment unit 233 should represent the multi-nucleus wave function by $3n_{qn}n_{nucl}$ first qubits 321n (apart from nuclear displacements) in the same way that the multi-electron wave function is represented by $3n_{qe}n_{e}$ second qubits 321e. In this case, the kinetic part $\exp(-iT\Delta t)$ of the real-time evolution operator can include contributions from the nuclei as well as the electrons. Both the kinetic part of the electrons and the kinetic part of the nuclei can be implemented on a quantum circuit by using the quantum Fourier transform. Thus, the ground state of a purely quantum mechanical system consisting of electrons and nuclei can be obtained by the probabilistic imaginary-time evolution method. The lowest energy state obtained in this case is at absolute zero.

Based on the above information processing method, it is also possible to obtain the lowest energy state at a finite temperature by calculating probabilistic imaginary time evolution while treating both electrons and nuclei as quantum mechanical particles. In this case, as the energy, we only need to obtain the electronic state and nuclear configuration with the lowest free energy at a finite temperature.

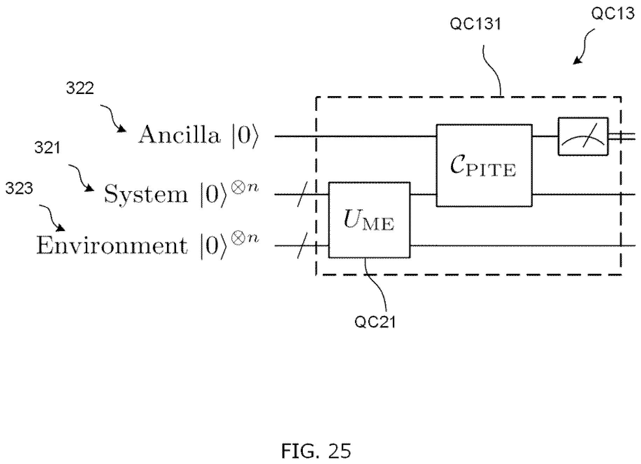

Below are details of a variant of the information processing method for identifying the configuration of the nuclei at finite temperatures. FIG. 25 shows an example of a second quantum gate operation QC13 that generates Gibbs states for finite temperature calculations. In this variant, the qubit 320 further includes an environment bit 323. The environment bit 323 is used to represent the interaction with the environment, which indicates that the system is at a finite temperature.

The multi-electron multi-nucleus wave function is represented using $3n_{qe}n_{e} + 3n_{qn}n_{nucl}$ computational qubits 321 (hereafter denoted as n computational qubits 321 for the sake of explanation). The second quantum gate operation QC13 (unit quantum circuit QC131 in detail) that generates the Gibbs state includes, in addition to the search quantum circuit C_PITE and the observation operation of the ancillary bit 322, a maximally entangled state generation circuit U_ME. The maximum entangled state generation circuit U_ME is configured to act on the computational qubits 321 (i.e., the first 321n and second 321e qubits) representing the target system, such as a molecular system, and the environment bit 323. The computational qubit 321, which represents the target system, is labeled "System," and the environment bit 323 is labeled "Environment.

<Maximally entangled state generation circuit U_ME>

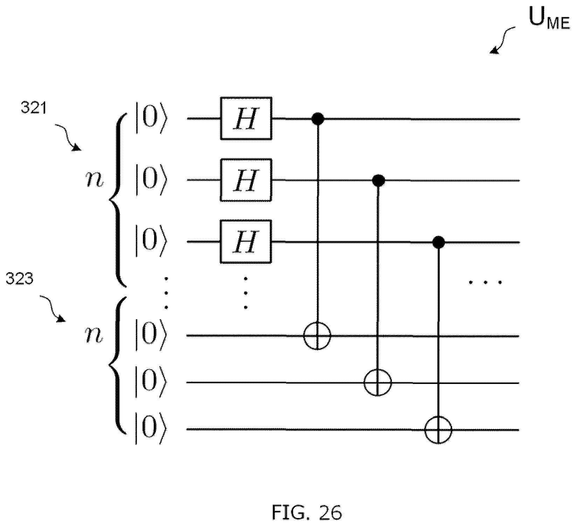

FIG. 26 shows an example of the maximally entangled state generator circuit U_ME.

The maximally entangled state generation circuit U_ME is configured so that the control NOT gates with the computational qubit 321 as the control bit and the environmental bit 323 as the target bit are applied after the Adamar gates H act on the computational qubits 321 and the ancillary bits 322. The search quantum circuit C_PITE for quantum mechanical systems consisting of electrons and nuclei can be constructed in the same way as described above.

By setting the imaginary time step $\Delta\tau$ to $1/(2k_B\times T)$ ($k_B$ is Boltzmann's constant and T is temperature), the n computational qubits 321 for the target system turn into the Gibbs state $\exp(-H_{en}/(k_B T))$ if the state of ancillary bit 322 is observed as $|0\rangle$. $H_{en}$ is the Hamiltonian of the target system. Furthermore, it is possible to derive a relation linking the probability of obtaining such an observation result to the distribution function Z of the target system. Thus, Z can be calculated from the number of times that $|0\rangle$ is obtained out of a large number of ancillary bit 322 measurements. The free energy can be calculated from the result and the well-known thermodynamic relation $\exp(-F/(k_B T)) = Z$ for the free energy F. By comparing the free energies of each configuration candidate, the optimal nuclear configuration can be identified.

In the above information processing, the number of qubits used to represent the electronic state by dividing each direction of three-dimensional space at equal intervals was set to $n_{qe}$ and the number of qubits for nuclear displacement was set to $n_{qn}$, but the number of computational qubits 321 assigned for each degree of freedom can be changed arbitrarily. For example, these conditions may be relaxed to adopt different qubit numbers $n_{qe\mu}$ and $n_{qn\mu}$ ($\mu$ = x, y, z)

depending on the direction. The multi-qubit state |Ψ> used in this case consists of 3 (n_e (n_qex + n_qey + n_qez) + n_nucl (n_qnx + n_qny + n_qnz)) qubits.

In the above information processing, although the candidate generation unit 232 generates, in activity A3, a plurality of configuration candidates of the nuclei from the reference position R_v0 by combining the displacement ΔR_ν (ν = 0, 1,..., n_nucl-1) of the ν-th nucleus from the reference position R_v0 and the acquisition unit 231 acquires the generated configuration candidates of the nuclei, the way to utilize these two units is not limited to that. For example, the acquisition unit 231 may acquire a plurality of configuration candidates of the nuclei prepared in advance by the user. Furthermore, configuration candidates of the nuclei may include configurations that are discontinuous with each other, which are difficult to produce by combining displacements ΔR_ν at regular intervals with the reference position R_v0.

The acquisition unit 231 may acquire the Hamiltonian H itself, including the interaction V at activity A2. In this case, the acquisition unit 231 does not need to generate the Hamiltonian H from the interaction V. The acquisition unit 231 may also accept a Hamiltonian H setting from the user.

The above information processing system 1 may be realized by a program that causes a computer to function as the information processing system 1. The program may be downloadable from a server, the program may be executed or distributed on a cloud computer, or such a program may be distributed by storing it on a non-volatile or volatile storage medium.

In the above embodiment, although the unit quantum circuit QC131 is implemented with the Hamiltonian H~ explicitly containing the nuclear coordinate R_ν, it can be also implemented in the similar way with the Hamiltonian H that does not handle the nuclei explicitly.

The processor 23 does not have to have the above quantum computation by the computational quantum circuit QC1 performed using the quantum computer 3 in reality. For example, the processor 23 may convert the quantum computation algorithm specified by the generated computational quantum circuit QC1 into a computation algorithm that can be executed by a classical computer, and then allow the classical computer to execute the computation algorithm. For example, the processor 23 may convert the quantum operation represented by the computational quantum circuit QC1 into a computational algorithm that can be performed by a classical computer by replacing it with a matrix computation problem.

Some of the processing of the processor 23 may be converted into a computational algorithm that can be executed by a quantum computer and then have the quantum computer 3 execute the computational algorithm.

The quantum computer 3 may function as a quantum sensing apparatus. For example, in this case, the computational quantum circuit 5 is represented by unitary operations for initial state generation and free-time evolution.

The information processing unit apparatus 2 may be a classical or quantum computer, or a combination of the two.

The above information processing system 1 can be applied to various types of information processing related to quantum computation, such as quantum measurement and quantum communication.

The mode of the above embodiments is not limited to the information processing system 1, but may be an information processing method or an information processing program. The information processing method includes each step of the information processing system 1. The information processing program causes at least one computer to perform each step of the information processing system 1.

6. As for an example of the first information processing in the case of using the adiabatic time evolution method as the ground state calculation method (the third information processing),
the above ground state calculation method may include the so-called adiabatic time evolution method. This section describes an example of the first information processing performed using the adiabatic time evolution method as the ground state calculation method. The information processing described in this chapter is sometimes referred to as the third information processing to distinguish it from the first and second information processing described above. For convenience of explanation, the explanation may be omitted below for the parts or processes common to the first information processing among the third information processing. The following process can be used in the second information processing as appropriate. The third information processing flow is similar to the first information processing flow shown in FIG. 6. Therefore, the differences between the first and third information processing in each activity are explained here.

6.1.1. As for the system to which the third information processing is applied,

first, we describe the system to which the third information processing is applied. This system is defined, for example, by the various information contained in the substance information IF1. The system is only an example, and the third information processing can be applied to any system.

In the present embodiment, the system is composed of a total of (N_{el} + N_{ion}) particles, including N_{el} electrons and N_{ion} nuclei. In the present embodiment, for the sake of explanation, the charge of each nucleus is assumed to be equal. Therefore, in the present embodiment, nuclei and ions are treated almost identically, and therefore particles corresponding to nuclei are described as ions. The Hamiltonian H_{final} of the system in the present embodiment is set as follows, for example, based on number 1.

[number 25]

$$H_{final} = \sum_{i=1}^{N_{el}} \left( -\frac{1}{2} \nabla_i^2 \right) + \sum_{i,j} v_{ee} \left( r_i - r_j \right) + \sum_{i,I} v_{ei} \left( r_i - R_I \right) + \sum_{I,J}^{N_{ion}} v_{ii} \left( R_I - R_J \right)$$

Here, N_{el} corresponds to n_{e} in number 1. N_{ion} corresponds to n_{nucl} in number 1. Each of i and j is an index representing an individual electron in the system, corresponding to l and l' in number 1, respectively. Each of I and J is an index for an individual nucleus in the system, corresponding to v and v' in number 1, respectively. r is the electron position operator. R is the position operator of the nucleus. The first term is the electron kinetic energy term, denoted T_{el}.

The first term is the electron momentum energy term, and is written as T_{el} in the following. The second term represents the electron-electron interaction and is represented by the interaction operator v_{ee}. In the following, the second term is denoted as V_{ee}. The third term represents the electron-nucleus interaction and is represented by the interaction operator v_{ej}. In the following, the third term is denoted as V_{ei}. The fourth term represents the interactions between nuclei and is represented by the interaction operator v_{ii}. In the following, the fourth term is denoted as V_{ii}. These interaction operators may include the charge Z of the nucleus as a parameter, as appropriate.

The indices i, j, I, and J are attached to the momentum and position operators in the equation. The i in the third term v_{ei} and the fourth term v_{ii} is a symbol indicating that it relates to a nucleus, which is different from the i used as an index to identify particles.

Based on such a system, the candidate generator 232 generates configuration candidates of the atomic nucleus in the candidate generation process in the same manner as in the first information processing, and assigns the configuration candidates to the first qubit 321n in the assignment process. The qubit state represented as a result of the assignment is represented, for example, as follows

[number 26]

$$|\Psi\rangle = \sum_{\{R_I\}} |\psi_{\mathrm{el}}(\{R_I\})\rangle \otimes |\{R_I\}\rangle$$

|Ψ> denotes the qubit state of the entire system consisting of N_{el} electrons and N_{ion} nuclei. | ψ_{el}({R_I})> denotes a partial qubit state (i.e., a second qubit state) composed of N_{el} electrons. |{R_I}> indicates a partial qubit state (i.e., the first qubit state), which is composed of N_{ion} nuclei. In the present embodiment, for convenience of explanation, n_{ion} first qubits 321n are used to represent the states of n_{ion} nuclei, and n_{el} second qubits 321e are used to represent the states of n_{el} electrons. This allows the first qubits 321n to represent $2^{(n_{ion})}$ different nuclear states, and the second 321e qubits to represent $2^{(n_{el})}$ different electron states.

6.2.2 Quantum Circuit Generation Process

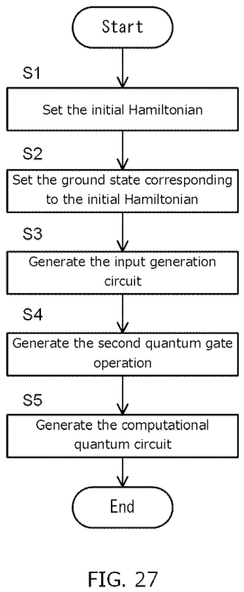

Next, the quantum circuit generation process (Activity A5), which is performed in the third information processing step, is described. FIG. 27 is a flowchart of the quantum circuit generation process in the present embodiment.

First, in step S1, processor 23 sets the initial Hamiltonian H_{initial}. The initial Hamiltonian H_{initial} may be set automatically based on the acquired Hamiltonian H_{final} of the system, set independently of the Hamiltonian H_{final} of the system, or set arbitrarily by the user. The initial Hamiltonian H_{initial} of the present embodiment is expressed, for example, by the following formula.

[number 27]

$$H_{initial} = T_{el} \otimes I_{ion} - J_x I_{el} \otimes \sum_{l=1}^{n_{ion}} X_l$$

In the above initial Hamiltonian H_{initial}, the operators T_{el} and J_x*I_{el} acting on the electronic state (i.e. the second qubit 321e) represents the Hamiltonian of the electron in free space, and X_1, which acts on the nucleus (i.e. the first qubit 321n), represents the X-gate operation on the 1-th first qubit 321n. I_{el} and I_{ion} are identity operators that act only on the electronic and nuclear portions of the 321 computational qubits, respectively.

Next, in step S2, processor 23 sets the ground state corresponding to the set initial Hamiltonian H_{initial}. The ground state |Ψ_{init}> of the initial Hamiltonian H_{initial} described above is expressed, for example, as follows.

[number 28]

$$\left| \Psi_{\mathrm{init}} \right\rangle = \left| \psi_{\mathrm{init}} \right\rangle \otimes \left| + \right\rangle^{\otimes n_{\mathrm{ion}}}$$

$$\left| + \right\rangle = \frac{\left| 0 \right\rangle + \left| 1 \right\rangle}{\sqrt{2}}$$

|Ψ_{init}> is also referred to as the input state to the second quantum gate operation QC13. |ψ_{init}> is the ground state of the Hamiltonian for the entire N_{el} electron system when interactions between particles are neglected and only the kinetic energy T_{el} of electrons is considered. |+> is also an example of a superposition of the |0> state and the |1> state of each nucleus constituting the system. In the present embodiment, |+> is the state in which the |0> state and the |1> state of each nucleus are superimposed with equal weight. The weight of each state is arbitrary when making a superposition of the |0> state and the |1> state of each nucleus. For the sake of explanation, the ground state of the initial Hamiltonian H_{initial} is hereafter referred to as the initial ground state.

Next, in step S3, the processor 23 generates the input generation circuit QC10 based on the ground state corresponding to the initial Hamiltonian H_{initial}. The method of generating the input generating circuit QC10 is arbitrary, but processor 23 can generate the input generating circuit QC10 by, for example, identifying the input generating circuit QC10 corresponding to the initial Hamiltonian H_{initial} and its ground state based on the results of previous calculations.

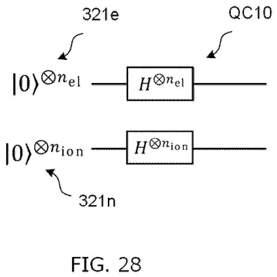

FIG. 28 shows an example of the input generation circuit QC10 in the present embodiment. The input generation circuit QC10 is configured to perform an Adamar gate operation on each of the first qubits 321n and second qubits 321e. |ψ_{init}> is obtained by performing an Adamar operation on each of the second qubits 321e in the |0> state. The |+> state is obtained by performing an Adamar operation on each of the first qubits 321n in the |0> state. Therefore, by inputting the initial state |000...00> of the computational qubits 321 to the above input generation circuit QC10, the input state |ψ_{init}> can be converted to the ground state of the initial Hamiltonian H_{initial}.

The input generation circuit QC10 is an example of the first quantum gate operation QC11, which is also an example of the reference quantum gate operation QC12. For example, the series of Adamar gate operations acting on the first qubit 321n in the input generation circuit QC10 corresponds to the first quantum gate operation QC11, which superposes the states of the first qubit 321n. In addition, since a series of Adamar gate operations acting on the second qubit 321e can generate a superposition state of reference electronic states for each configuration candidate of the nucleus included in the superposition state, the series of Adamar gate operations correspond to the reference quantum gate operation QC12.

Thus, by performing the first quantum gating operation QC11 (input generation circuit QC10) on the computational qubit 321, which is the initial state, the qubits of the computational qubit 321 take the eigenstate (including ground state) of the predefined initial Hamiltonian H_initial}. Processor 23 can be said to assign states to the first 321n and second 321e qubits so that just prior to the second quantum gating operation, the state represented by the first and second qubit is an eigenstate of the initial Hamiltonian H_{initial}.

The form of the initial Hamiltonian H_{initial} is arbitrary, but one whose ground state can be calculated analytically, for example, one for which an exact solution exists, is preferred. This improves the accuracy of the calculation results because the error between the input state and the ground state input to the computational quantum circuit QC1 is smaller.

As shown in FIG. 27, after processing step S3, the process proceeds to step S4, where processor 23 generates a second quantum gate operation QC13 based on the acquired Hamiltonian of the system (see number 26 above). The second quantum gate operation QC13 in the present embodiment is constructed to specify the adiabatic time evolution for the generated input state |Ψ_{init}>.

Processor 23 then sets up the second quantum gate operation. In this case, the second quantum gate operation QC13 is a gate operation corresponding to the time evolution of the system by the virtual Hamiltonian H(t), which changes almost adiabatically from the initial Hamiltonian H_{initial} to the objective Hamiltonian H_{final} using at least one real-time evolution operator. The virtual Hamiltonian H(t) is expressed, for example, by the following equation.

[number 29]

$$H(t) = T_{el} \otimes I_{ion} + A_1(t)V_{ee} \otimes I_{ion} + A_2(t)V_{ei} + A_3(t)I_{el} \otimes V_{ii} - \left(1 - A_4(t)\right)J_x I_{el} \otimes \sum_{l=1}^{n_{ion}} X_l$$

The first term on the right side indicates the kinetic energy of the electron. The second term on the right side indicates the electron-electron interaction. The third term on the right-hand side indicates the electron-nucleus interaction. The fourth term on the right-hand side indicates the interactions between nuclei. The fifth term on the right-hand side, together with the first term on the right-hand side, represents the initial Hamiltonian H_{initial} above. The coefficient A_i(t) (i = 1, 2, 3, 4) is a parameter that depends on time t to introduce each interaction almost adiabatically. The coefficient A_i (t) is 0 in the initial state (i.e., when t = 0) and 1 in the final state (when t = t_f) and is configured to increase with increasing t, for example.

Here, the time evolution operator of the system due to the contribution of the first term on the right-hand side of the number 29 is expressed as follows using the Centered Quantum Fourier Transform hereafter referred to as CQFT).

[number 30]
$$\mathrm{CQFT} \cdot U_{\mathrm{kin}} \cdot \mathrm{CQFT}^\dagger = \exp\left[-iT_{\mathrm{el}}\Delta t\right]$$

In addition, by each of the time evolution operators of the system due to the contributions of the second, third, and fourth terms on the right-hand side of number 29, the time evolution of the qubit state |Ψ> is represented with the time width Δt, which represents the unit of time evolution, as follows.

[number 31]
$$U_{\mathrm{ee}}^{(k)} = \exp\left[-iA_1(k\Delta t)V_{\mathrm{ee}}\Delta t\right]$$

$$U_{\mathrm{ei}}^{(k)} = \exp\left[-iA_2(k\Delta t)V_{\mathrm{ei}}\Delta t\right]$$

$$U_{\mathrm{ii}}^{(k)} = \exp\left[-iA_3(k\Delta t)V_{\mathrm{ii}}\Delta t\right]$$

$$R_x^{\otimes n_{ion}}(\theta_k) = exp\left[i\Delta t\left(1 - A_4(k\Delta t)\right)J_x I_{el} \otimes \sum_{l=0}^{n_{ion}} X_l\right]$$

$$\theta_k = -2\left(1 - A_4(k\Delta t)\right)J_x \Delta t$$

R_x^{*n_{ion}}(θ_k) (where * denotes the Kronecker product) is a rotary gate operation that rotates the respective states of the first qubits 321n by a rotation angle θ_k with the x-axis as the rotation axis. For convenience of explanation, the operator that represents the time evolution by the virtual Hamiltonian is hereinafter referred to as the virtual time evolution operator. The expressions for each interaction are not limited to those listed above. For example, it is possible to adopt an expression equivalent to the above formula by applying any transformation to each coefficient A_i or parameter θ_k, such as sign inversion, constant multiplication, constant addition or subtraction, etc. to the above formula. The qubit state | Ψ(nΔt) > that is time evolved by n×Δt from the initial state | Ψ(0) > by such a virtual time evolution operator can be expressed, for example, within the first-order Suzuki Trotter expansion as follows.

[number 32]
$$|\Psi(n\Delta t)\rangle = \prod_{k=n}^{1}\left[R_x^{\otimes n_{ion}}(\theta_k) \cdot CQFT \cdot exp\left[-i\overline{K}\Delta t\right] \cdot CQFT^\dagger \cdot exp\left[-iA_3(k\Delta t)V_{ii}\Delta t\right]\right.$$

$$\left. \cdot exp\left[-iA_2(k\Delta t)V_{ei}\Delta t\right] \cdot exp\left[-iA_1(k\Delta t)V_{ee}\Delta t\right]\right]|\Psi(0)\rangle$$

The expression in number 32 may be , for example, combined with higher-order Suzuki Trotter expansions, and is arbitrary, as long as it represents the virtual time evolution rigorously or approximately.

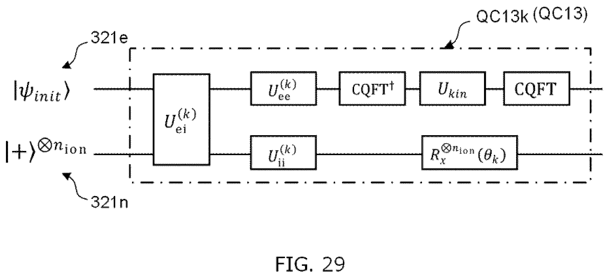

FIG. 29 shows an example of a quantum circuit representing a virtual time evolution operator as the second quantum gate operation QC13. Since the second quantum gate operation QC13 varies according to an integer k (k = 1, 2,...) corresponding to the number of iterations, the second quantum gate operation QC13 corresponding to said k is sometimes denoted as QC13k. Each of the first qubits 321n is in the | +> state and each first qubit state is superposed. The second quantum gate operation QC13 is operated from k = n to k = 1, leading to the final overall qubit state of the computational qubits 321, which is the final state | $\Psi(n\Delta t)$ > expressed in number 32.

In the above equation, exp[-iK$\Delta$t] is defined as exp[-iK$\Delta$t] = U_{kin}. Thus, processor 23 generates a virtual state by acting on the superposition state of the first qubit state with a virtual time evolution operator that includes the time width $\Delta$t of the time evolution as a parameter. Each coefficient A_i is set to become 1 when the end time t_f = n$\Delta$t.

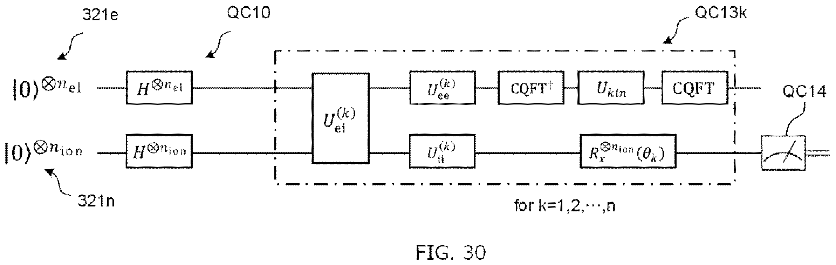

The process then proceeds to step S5, where processor 23 generates a computational quantum circuit QC1 based on the above input generation circuit QC10 and the second quantum gate operation QC13. FIG. 30 shows an example of the computational quantum circuit QC1 in the present embodiment.

The computational quantum circuit QC1 in the present embodiment includes an input generation circuit QC10 and a second quantum gate operation QC13k (k = 0, 1, 2,..., n). First, the computational quantum circuit QC1 is configured to act the input generation circuit QC10 on each of the initialized first qubit 321n and second qubit 321e. This causes the computational qubits 321 to be the ground state or close to the ground state of the initial Hamiltonian H_{initial}.

The computational quantum circuit QC1 is configured to perform the second quantum gate operation QC13k in sequence from k=0 to n after the input generation circuit QC10. This causes the ground state of the initial Hamiltonian H_{initial} to gradually adiabatically evolve in time with a time width $\Delta$t, leading to the final state | $\Psi(n\Delta t)$>.

The computational quantum circuit QC1 is then configured to perform the observation operation QC14 on the first qubit 321n. This makes it more likely to observe the state of the first qubits 321n, where the energy of the entire substance represented by the computational qubits 321 is low. Thus, processor 23 can identify the most stable nuclear configuration by aggregating such observations.

The system to which the third information processing is applied is not limited to systems containing multiple types of particles, such as nuclei and electrons, but can also include systems containing a single type of particle, such as systems containing only nuclei. For example, in the case of the third information processing described above for a system consisting only of nuclei, the generated computational quantum circuit QC1 includes an input generation circuit QC10 and a second quantum gate operation QC13 at as in the system containing electrons described above. The input generation circuit QC10 in this case should only include the gate operation acting on the first qubit 321 in the input generation circuit QC10 for the system containing electrons. The second quantum gate operation QC13 should include U_{ii}^{(k)} and R_x^{*n_{ion}}($\theta$_k) which are same as those for the system containing electrons.

6.3 Simulation results when the adiabatic time evolution method is employed

In this section, we describe simulation results when the adiabatic time evolution method is employed as the ground state search method. In other words, this section describes the simulation results using the computational quantum circuit QC1 by the third information processing described above. The simulation results are only an example to show the usefulness of this information processing and do not limit the interpretation of the technical concept of the information processing described above.

The substance to be calculated in this simulation is molecular hydrogen (H2), and the substance information IF1 includes the atomic species contained in the hydrogen atom (i.e., two Hs) and the bond length d between the hydrogen nuclei (an example of nuclear configuration). In the present embodiment, the bond length d is set to 2. For computational convenience in the present embodiment, the bond length d is a dimensionless quantity normalized by the Bohr radius.

The potentials acting on each particle constituting the molecular hydrogen in the present embodiment are set as follows.

$$[\text{number } 33]$$

$$V_H(x) = \frac{1}{\sqrt{x^2 + 1}}$$

Here, x indicates the coordinate corresponding to the position of the particle in a one-dimensional coordinate system with the midpoint of the two nuclei as 0 and the x-axis in the direction toward each nucleus.

Next, the candidate generator 232 generates candidates for other nuclear configurations for the acquired bond length d. In the present embodiment, in addition to d = 2, d = 4, 6, and 8 are generated as candidates.

Next, the assignment section 233 assigns the generated candidate nuclear configurations to the qubit states of the first qubit 321n. In this embodiment, two first qubits 321n are used to represent the four candidate nuclear configurations. Specifically, the assignment section 233 assigns a candidate nuclear configuration for each of the first qubit states, such that an configuration with d = 2 indicates |00>, an configuration with d = 4 indicates |01>, an configuration with d = 6 indicates | 10>, and an configuration with d = 8 indicates | 11>.

Processor 23 then performs the third information processing based on this information to generate the computational quantum circuit QC1. The simulation results described below are obtained by having a classical computer simulate the computation by the computational quantum circuit QC1 generated in this way. In the present embodiment, the total number of steps n is 10000, and the elapsed time t_f in which adiabatic time development has taken place from the initial state to the final state is $\Delta$t*10000.

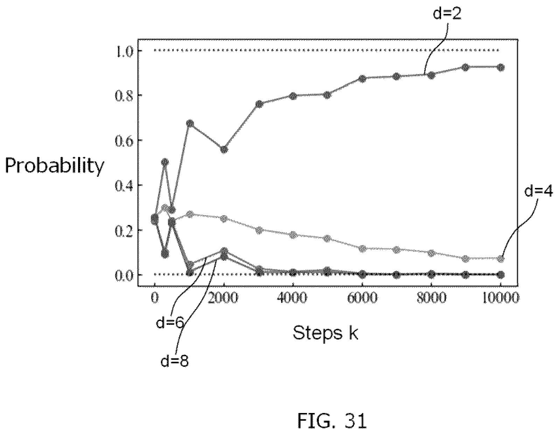

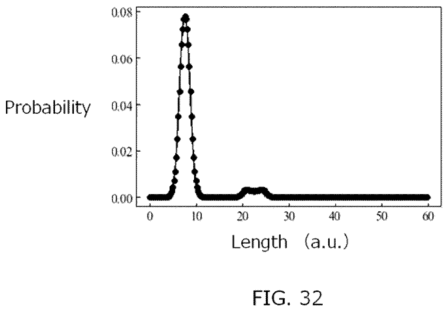

FIG. 31 shows the relationship between the observation probability of each candidate and the number of steps as a simulation result when the adiabatic time evolution method is employed as the ground state search method. FIG. 32 shows the components in the wave function in the final state. The abscissa of FIG. 32 is a parameter with a dimension of length, which can be mapped to the distance between nuclei. In FIG. 32, the region between 0 and 15 corresponds to d = 2, between 15 and 30 to d = 4, between 30 and 45 to d = 6, and between 45 and 60 to d = 8.

As shown in FIG. 31, the observation probability of d=2 increases with each increase in the number of steps. As shown in FIG. 32, the observation probability of d = 2 is more than 9 times higher than the probability of the other states in the final state. This suggests that the configuration of the nuclei with d = 2 is the most stable structure.

If the energy E_gs of the ground state of each candidate is calculated using the conventional classical algorithm, E_gs = -0.8107 for d = 2, E_gs = -0.7497 for d = 4, E_gs = -0.6932, and E_gs = -0.6789 for d = 8. Therefore, d = 2 is shown to be the most stable state. These results are consistent with the theoretical conclusion that the configuration corresponding to d = 2 is the lowest energy configuration, suggesting the validity of this calculation method.

6.4. information processing (fourth information processing) to compute the ground state from the final state

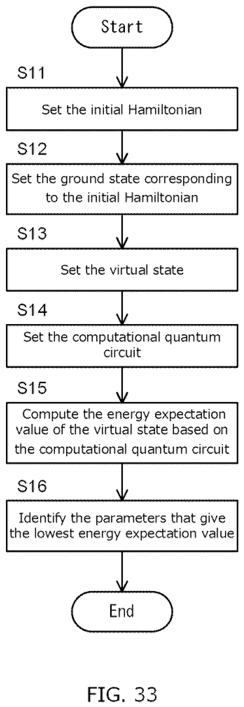

Next, we describe the information processing for calculating the ground state of the system from the ground state of the number 32 initial Hamiltonian H_{initial}. The information processing is hereinafter referred to as the fourth information processing. FIG. 33 is a flowchart of the fourth information processing flow.

First, processor 23 sets the initial Hamiltonian H_{initial} in step S11. The details of step S11 are the same as in step S1.

Next, processor 23 sets the ground state corresponding to the initial Hamiltonian H_{initial} in step S12. The details of step S12 are the same as in step S2.

Next, processor 23 sets the virtual state |$\Psi$_{QAOA}> based on the Hamiltonian H_{final} (see number 25) of the system in step S13. The virtual state |$\Psi$_{QAOA}> is set based on the final state | $\Psi$(n$\Delta$t)>= |$\Psi$(t_f)> (see number 32) when the virtual Hamiltonian H(t) described above is adiabatically time evolved from the initial Hamiltonian H_{initial} to the Hamiltonian H_{final} of the system. For example, the virtual state |$\Psi$_{QAOA}> is obtained by parameterizing the quantity pertaining to the time width $\Delta$t contained in each of the components corresponding to each energy term in |$\Psi$(n$\Delta$t)> (e.g., each component expressed by number 31). In the present embodiment, the virtual state|$\Psi$_{QAOA}> is represented by using each unknown variable $\alpha$_k, $\beta$_k, $\gamma$_k, $\delta$_k, $\theta$_k as follows.

[number 34]

$$|\Psi_{\mathrm{QAOA}}\rangle \equiv \prod_{k=1}^{p} \left[ R_x^{\otimes n_{\mathrm{ion}}}(\theta_k) \cdot \mathrm{CQFT} \cdot \exp\left[-i\bar{K}\alpha_k\right] \cdot \mathrm{CQFT}^\dagger \cdot \exp\left[-iV_{\mathrm{ii}}\beta_k\right] \right.$$
$$\left. \cdot \exp\left[-iV_{\mathrm{ei}}\gamma_k\right] \cdot \exp\left[-iV_{\mathrm{ee}}\delta_k\right] \right] |\Psi(0)\rangle$$

Each unknown variable $\alpha$_k, $\beta$_k, $\gamma$_k, $\delta$_k, and $\theta$_k are coefficients that parameterize the contribution of each interaction in the Hamiltonian H_{final} of the system. $\alpha$_k is the unknown variable corresponding to U_{kin}. $\beta$_k is the unknown variable corresponding to V_{ii}. $\gamma$_k is the unknown variable corresponding to V_{ei}. $\delta$_k is the unknown variable corresponding to V_{ee}. $\theta$_k indicates the angle of rotation with the above x-axis as the axis of rotation. |$\Psi$(0)> is the initial state (i.e., the ground state corresponding to the initial Hamiltonian).

Next, processor 23 generates a computational quantum circuit to generate the virtual state | $\Psi$_{QAOA}> from the initial state |$\Psi$(0)> by setting each unknown variable $\alpha$_k, $\beta$_k, $\gamma$_k, $\delta$_k, $\theta$_k as appropriate in step S14. The quantum circuit is specified by the operator acting on the initial state | $\Psi$(0) > in the above number 34. Here, multiple sets of each unknown variable {$\alpha$_k, $\beta$_k, $\gamma$_k, $\delta$_k, $\theta$_k} are set, and a computational quantum circuit is generated for each set.

Next, processor 23 calculates the energy expectation value of the virtual state |$\Psi$_{QAOA}> based on the generated computational quantum circuit in step S15. The relevant energy expectation E is expressed as follows.

[number 35]

$$E = \langle \Psi_{QAOA} | H(t_f) | \Psi_{QAOA} \rangle$$

H(t_f) is the time when each coefficient A_i in the virtual Hamiltonian H(t) becomes 1. In other words, the above energy expectation E is the energy expectation of the virtual state for the final state of the virtual Hamiltonian due to time evolution.

Next, processor 23 identifies the unknown variables $\alpha\_k$, $\beta\_k$, $\gamma\_k$, $\delta\_k$, and $\theta\_k$ that have the minimum energy expectation value above in step S16. Thereby, processor 23, as the parameter determination unit, determines the parameters by minimizing the energy expectation value of the final virtual state of the virtual Hamiltonian due to time evolution. Identifying the most stable state of the substance specified by the substance information IF1 is attributed to identifying each unknown variable $\alpha\_k$, $\beta\_k$, $\gamma\_k$, $\delta\_k$, $\theta\_k$ such that the above energy expectation value E is minimized. If the energy expectation E for each of the multiple sets of unknown variables has been calculated, processor 23 determines the amount of change in the unknown variables $\alpha\_k$, $\beta\_k$, $\gamma\_k$, $\delta\_k$, and $\theta\_k$ based on such multiple energy expectation values E, and calculates the energy expectation value E corresponding to the set of unknown variables $\alpha\_k$, $\beta\_k$, $\gamma\_k$, $\delta\_k$, and $\theta\_k$ after reflecting the changes. If the change in the expected energy value due to this is less than or equal to the specified error tolerance, the virtual state $| \Psi\_{QAOA}\rangle$ represented by the final set of unknown variables $\alpha\_k$, $\beta\_k$, $\gamma\_k$, $\delta\_k$, $\theta\_k$ represents the most stable state of the substance (most stable structure structure). The most stable nuclear configuration is obtained by observing the state of the first qubit 321n of the virtual state $|\Psi\_{QAOA}\rangle$. The above identification of the most stable state may be calculated as an optimization problem by a classical computer or by using a quantum computer as appropriate. The method of minimizing the above energy expectation E is arbitrary and may be, for example, various methods used for classical variational problems.

The initial Hamiltonian H_{initial} is not limited to the form expressed in number 27 and may further include an initial potential V_{init}. The initial potential V_{init} is a pre-given potential energy, for example, the potential of an electron system. If such an initial potential exists, the initial Hamiltonian H_{initial} is expressed as follows

[Number 36]

$$H_{initial} = (T_{el} + V_{init}) \otimes I_{ion} - J_x I_{el} \otimes \sum_{l=1}^{n_{ion}} X_l$$

When such an initial Hamiltonian H_{initial} is set, processor 23 can set the virtual Hamiltonian H(t) as follows.

[number 37]

$$H(t) = T_{el} \otimes I_{ion} + A_1(t)V_{ee} \otimes I_{ion} + A_2(t)V_{ei} - \left(1 - A_4(t)\right) J_x I_{el} \otimes \sum_{l=1}^{n_{ion}} X_l + A_5(t)V_{ext}$$

$$\otimes I_{ion} + \left(1 - A_6(t)\right) V_{init} \otimes I_{ion}$$

Coefficients A_5 and A_6 are coefficients that represent the contribution of each energy, similar to A_1, A_2, A_3, and A_4 above.

In this case, $|\psi\_{init}\rangle$ expressed in number 28 etc. is the ground state of the Hamiltonian of the entire N_{el} electron system when the kinetic energy T_{el} of electrons and the initial Hamiltonian V_{init} are considered.

The quantum circuit for generating $|\psi\_{init}\rangle$ does not have to be specified to perform Adamar gate operations on each of the first qubits 321n and second qubits 321e, and the initial Hamiltonian H_{initial} can be determined according to the number of electrons in the system as appropriate.

7. On the generation of the computational basis

In this chapter, another example of generation of the computational basis assigned to the computational qubits 321 when performing each of the above information processing is described, along with the flow of that information processing. The particles in this form are the nuclei that make up substance and are classical point particles, but they may be any interacting particle, including but not limited to electrons, quasiparticles, aggregates, etc. For convenience of explanation, the information processing to generate the computational basis is hereafter referred to as the fifth information processing. The fifth information processing is used, for example, as the processing from Activity A2 to Activity A4 in the first or second information processing (i.e., from acquisition of substance information IF1 to execution of the assignment process).

7.1. As for flow of the fifth information processing

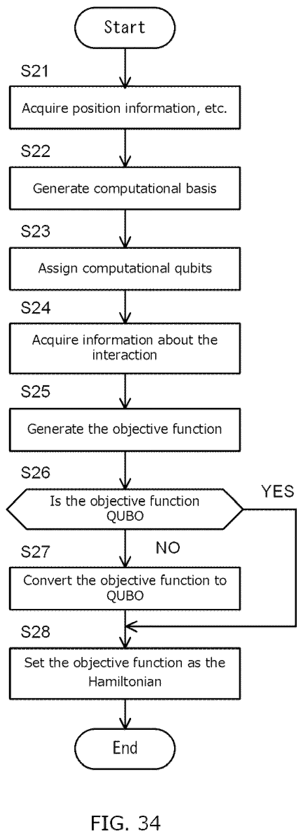

This section describes the fifth information processing flow. FIG. 34 is a flowchart showing the fifth information processing flow.

First, in step S21, the acquisition unit 231 acquires position information regarding the L arrangement candidates in a certain space and classification information regarding k types of classification of N particles to be placed in the candidate arrangements. The position information indicates, for example, the coordinates at which each of the N particles can be placed. Position information is also the position where particles can be placed in a certain space, which can also be called a specific position. The specific position is represented, for example, by a single coordinate point where particles can be present. In other words, the acquisition unit 231 acquires information about a plurality of specified positions. In particular, the position information indicates the coordinates at which each of the N particles can be placed, regardless of classification. The arrangement candidates are generated, for example, in a manner similar to the information processing described above. For convenience of explanation, the space where arrangement candidates are set up is hereinafter referred to as the target space. Specifically, the arrangement candidates can be represented as L grid points by dividing the target space into equally spaced sections. Each of these grid points is configured to allow N particles to be placed. In the present embodiment, the target space is a three-dimensional real space, and at each of the grid points, in the system representing the substance, zero or one particle is placed, so that multiple particles are not placed in the same candidate location at the same time. The target space has $N\_d$ arrangement candidates (grid points) per dimension. Therefore, the target space has a total of $N\_d^{\wedge} 3$ grid points as arrangement candidates. The arrangement candidates can also be said to be the number of divisions of the target space.

Classification in classification information is information to distinguish between different types of particles, and in the present embodiment, includes the atomic species of the particles that make up the substance, such as hydrogen atoms, oxygen atoms, and carbon atoms. The classification is not limited to this and can include classification by any state, such as ionic valence, isotopes, etc. For example, the classification may represent the type of particles that make up an atom, such as electrons, nuclei, and protons. The classification is not limited to representing a single particle, but can also be information that distinguishes micelles or other composite particles. Classification information can be said to represent the classification of particles.

Next, in step S22, processor 23, as a basis generator, generates a computational basis represented by at least k x L computational qubits 321 based on the acquired position information. The computational qubits 321 include at least L position qubits corresponding to each of the candidate arrangements, set for each of the k classifications. In the present embodiment, processor 23 ,assuming that each of the k types of particles can be placed in L common candidate positions, generates k x L computational bases. In this embodiment, the first qubits 321n function as position qubits.

Next, in step S23, the assignment unit 233 assigns the state of the computational qubits 321 to each of the generated computational bases. This allows the computational basis to be constructed so that it is possible to express which arrangement candidate each particle is placed in, for each type.

For example, if the classification is information that distinguishes four atomic species S, the qubit state |Ψ> of the computational qubit 321 is expressed as follows using the partial qubit state |S_l> of computational qubit 321 corresponding to the ground state of each atomic species S_l (l = 1, 2, 3, 4).

[number 38]

$$|\Psi\rangle = |S_1\rangle \otimes |S_2\rangle \otimes |S_3\rangle \otimes |S_4\rangle$$
$$|S_l\rangle = |01010\dots 0\rangle$$

Each of the computational bases is associated with L partial computational qubits 321 assigned to each atom species S_l, where the state of each qubit of L partial computational qubits 321 takes |0> or | 1>. For example, the computational basis corresponding to the state in which the atomic species S_l is located at the mth grid point of the L grid points is assigned to a qubit state such that the mth computational qubit 321 of the L computational qubits corresponding to the atomic species S_l has the state | 1>. In other words, the assignment unit 233 assigns at least one computational qubit to each of the specified positions for each particle classification. According to this configuration, the state of a complex system in which particles of multiple classifications are mixed can be represented with a smaller number of computational qubits 321.

The mode of the computational basis is arbitrary and is not limited to those represented by at least $k \times L$ computational qubits 321. In other words, the assignment unit 233 assigns a group of computational qubits that includes at least one computational qubit for each of the specific positions. A computational qubit group is composed of computational qubits 321 and can represent at least two different states in total. In other words, a group of computational qubits is configured so that at least the first state and the second state are observable. The first state indicates the presence of a particle at the corresponding specific position. The second state indicates that no particles are present at the corresponding specific position. In the following, we describe the case where a group of computational qubits consists of a single computational qubit 321. The first state in this case, for example, corresponds to a certain computational qubit 321 qubit state of | 1>, and the second state corresponds to a certain computational qubit 321 qubit state of |0>. According to this configuration, the state of the particle in a specific position is represented as the state of the computational qubit 321, thus enhancing the interpretability of the quantum computation.

In the next step S24 in this embodiment, the acquisition unit 231 further acquires information about the interaction between the particles to be placed in the arrangement candidates (grid points) and information about the number of particles per classification. The interaction is determined at least by the positional relationship between particles and the classification of particles. For example, the interaction between a particle at the i-th grid point and a particle at the j-th grid point can be represented by J_{ij}. The origin of such interactions can be arbitrary, such as interatomic forces, Coulomb forces, weak forces, strong forces, etc. Such interactions include interactions of more than three bodies, such as empirical potentials.

Next, in step S25, processor 23 generates an objective function based on the computational basis and the interaction information. The objective function includes a first factor and a second factor. The first factor corresponds to the Hamiltonian of the system consisting of particles. The second factor is configured to output a larger value when the first number representing the number of particles of a certain classification represented by the computational qubit is different from the second number representing the acquired number of such particles, than when the first number is equal to the second number. For example, the objective function is expressed as H_int as follows

[Number 39]

$$H_{\mathrm{int}} = \sum_{i<j} J_{ij} x_i x_j + \sum_{k \in S} P_k \left( \sum_{l=1}^{N_{\mathrm{grid}}} x_{k,l} - N_k \right)^2$$

Here, S is a set of atomic species, e.g., S = {H, C, O, N}. J_{ij} is a constant that represents the magnitude of the interaction, e.g., the value of the interaction potential, with reference to the distance and the atomic species. k_1 to k_N_ {grid} are indices representing the qubits assigned to atom k. N_k is the number of atoms corresponding to the classification k (i.e., the kth atomic species). N_{grid} is the total number of arrangement candidates and is equal to N_d^3. N_k may be entered manually by the user or estimated by processor 23 or others based on various measurements on the substance. P_k is a penalty factor to specify the number of atoms corresponding to the classification k and is positive. In the above equation, the first number is $\Sigma x_{k,l}$ and the second number is N_k. For example, the second factor is represented by an even function of the difference between the first and second numbers. In the present embodiment, the second factor is represented by an even function of the difference between the first and second numbers. The first term on the right side of the above equation is an example of the first factor and represents a two-body interaction. The second term on the right side above is an example of a second factor, where P_k is a positive constant.

Thus, the objective function H_{int} should be expressed in Ising-type function or QUBO (quadratic unconstrained binary optimization) form. Therefore, in the present embodiment, in step S26, processor 23 determines whether the objective function H_{int} is in QUBO format. The method of such determination is arbitrary, but can be based, for example, on whether or not the Hamiltonian form contains terms of third order or higher.

If the result of the decision in step S26 is negative, the process proceeds to step S27, where processor 23 converts the objective function H_{int} to QUBO format. In particular, if the objective function H_{int} is represented by an Ising type function, there is at least one objective function in QUBO format corresponding to the objective function H_{int}. If the objective function is not convertible to QUBO format, processor 23 may notify the user that the objective function is not convertible to QUBO format and suspend the information processing . The process then proceeds to step S28. On the other hand, if the judgment result in step S26 is positive, there is no need to convert again to QUBO format, so processor 23 omits processing in step S27 and proceeds to step S28.

At step S28, processor 23 sets the objective function thus converted to QUBO format to the Hamiltonian H in the first or second information processing. Processor 23 may set the objective function to the virtual Hamiltonian H(t) when performing the third information processing.

The fifth information processing is then completed, and processor 23 generates the computational quantum circuit QC1 based on the set Hamiltonian and executes the calculation using the quantum computer 3. The said computational quantum circuit QC1 includes at least the first quantum gate operation QC11 and the second quantum gate operation QC13 as well as the first and third information processing above. Thereby, the quantum manipulation unit 234 performs a first quantum operation on the computational qubit 321 based on the computational quantum circuit QC1 to generate a first superposition state in which the states corresponding to the computational basis are superposed. Such a state can be implemented, for example, by an Adamar gate operations that convert the individual qubit states of the first qubits 321n to the |+> state.

Next, the quantum manipulation unit 234 performs a second quantum manipulation corresponding to the objective function on the first superposition state based on the relevant computational quantum circuit QC1 to generate a second superposition state that reflects at least the interaction between the particles with respect to the first superposition state. The manner in which the second quantum gate operation QC13 is generated is the same as that described in the first information processing, etc.

Next, the identification unit 235 performs the process of identifying the arrangement with relatively low energy among the arrangement candidates of particles based on the observation results for the computational qubits 321 with the generated second superposition state. The specific form of the process of identifying such information is the same as that used in the first information processing, etc.

According to the fifth information processing described above, for example, it is possible to simplify the quantum circuit in identifying the optimal arrangement in a non-degenerate system. Thus, it is easier to identify the optimal arrangement in a non-degenerate system.

7.2. Variation of the Fifth Information Processing

The fifth information processing is not limited to those described above and can be implemented, for example, by combining the following variants as appropriate.

In the fifth information processing described above, processor 23 generated a computational basis represented by k x L computational qubits 321, but it is not limited to this. For example, processor 23 may generate a computational basis consisting of n x k x L computational qubits, where n is an integer greater than or equal to 2, so that the computational basis may further be configured to represent the case of multiple particles in the same arrangement candidate . In other words, the first qubit state may be configured such that states other than |0> and | 1> are assigned for a single grid point. For example, if three atoms can be placed on one lattice point, the assignment unit 233 may assign two first qubits 321n for that grid point. For example, the assignment unit 233 assigns the ground state corresponding to each number of nuclei to the first qubit state, so that the two first qubits 321n is in the state |00> when the number of nuclei placed at the grid point is 0, the two first qubits 321n is in the state | 01> when the number of nuclei placed at the grid point is 1, the two first qubits 321n is in the state | 10> when the number of nuclei placed at the lattice point is 2, and the two first qubits 321n is in the state | 11> when the number of nuclei placed at the grid point is 3. In other words, the computational qubit group includes two or more computational qubits 321 and is configured so that at least one qubit state different from the first and second state can be observed. Each of the qubit states corresponds to the number of particles in the corresponding specific position. According to this configuration, the interpretability of quantum calculations for more diverse systems can be improved, because the state in which multiple particles exist in the same specific position can be assigned to the state of a computational qubit, as in boson systems, for example.

The first factor is not limited to the form of the first term of H_{int} described above. For example, the contribution of the m-body interaction is expressed as an m-order polynomial of the binary variable x_i. Thus, it may be written in the form of HUBO (Higher Order Unconstrained Binary Optimization) as follows.

[number 40]

$$\sum_i J_i^{(1)} x_i + \sum_{i_1,i_2} J_{i_1 i_2}^{(2)} x_{i_1} x_{i_2} + \cdots + \sum_{i_1,i_2,\cdots,i_m} J_{i_1 i_2 \cdots i_m}^{(m)} x_{i_1} x_{i_2} \cdots x_{i_m}$$

The second factor is not limited to the second term form of H_{int} described above. For example, the second number in the second factor does not have to be a fixed value for each classification, such as the atomic species S, and may be variable in the following form.

[Number 41]

$$\left( C_{k_1} \sum_{l=1}^{N_{grid}} x_{k_1,l} - C_{k_2} \sum_{l=1}^{N_{grid}} x_{k_2,l} \right)^2$$

This allows the ratio of the number of particles of each classification to be taken as a constraint when minimizing the objective function H_{int}, since the value of the second factor will increase when the ratio of the number of particles of classification k_2 to those of classification k_1 is not equal to C_{k_1}/C_{k_2}. For example, when determining the arrangement of nuclei in silicon dioxide SiO2, processor 23 sets C_{k_1} = 2 and C_{k_2} = 1 as the second factor. Here, classification k_1 represents Si and classification k_2 represents O.

If the atomic species contains positive or negative ions, processor 23 may set a penalty factor (second factor) representing the neutral condition of the substance by obtaining information on the valence of the species. The relevant penalty factor can be expressed in the same form as number 41 above. For example, when determining the arrangement of nuclei in sodium chloride NaCl, we can set C_{k_1} = C_{k_2} = 1 so that the valence of the Na$^+$ ion (+1) and the Cl- ion (-1) are balanced. Furthermore, if an aggregate of nuclei is ionized (e.g., a carboxylic acid in aqueous solution), and the valence of the entire aggregate is Q, the processor 23 should set the second factor in the following form.

[number 42]

$$\sum_{k \in S} \left( C_k \sum_{l=1}^{N_{grid}} x_{k,l} - Q \right)^2$$

The coefficient C_k is a value set according to the valence of each nucleus (ion). The first term in the above equation represents the sum of the valence of the arranged nuclei (ions), and if the value of the first term is different from Q, the value of the second factor will increase. Therefore, by introducing such a second factor, processor 23 can take into account a constraint of the valence of ions in the target space when minimizing the objective function H_{int}.

The second factor need only include at least one of the above-mentioned forms, e.g., it may include more than one. This second factor can be generalized using the following equation.

[number 43]

$$\sum_{m \in M} P_m \left( \sum_{k \in S} \left[ C_{k,m} \sum_{l=1}^{N_{grid}} x_{k,l} + M_{k,m} \right] + Q_m \right)^2$$

m is an index representing the constraint M in the second factor, e.g., charge neutrality condition, particle number constraint, composition ratio constraint, etc. k denotes each of the atomic species S. The conditions pertaining to the k, such as C_{k, m} and M_{k, m}, represent the conditions imposed on the k-th atomic species. Q_m is a number that represents the condition imposed on the entire target space, regardless of the type of atomic species, and is a generalization of Q in number 42 above. Q_m can be set to 0 by renormalizing to M_{k, m} as appropriate. The second factor may be set to not be included by setting P_m = 0.

The target space is not limited to 3D real space, but can be a space based on coordinates other than real space coordinates, such as spin space or reciprocal space. The dimension of the target space is not limited to 3, but can be a lower dimensional space such as 1 or 2, or a hyperspace of 4 or more dimensions. In other words, the target space can be any space that can be described mathematically as a field, including the presence or absence and state of particles. In other words, the fifth information processing described above is not limited to that performed for arrangement candidates of particles (i.e., candidate positional states), but can be applied to any candidate state. In short, the acquisition unit 231 acquires state information about the L candidate states in a certain state space and classification information about the k types of classification of the N particles. Each of the particles takes on one of the candidate states. Processor 23, as the basis generator, generates a computational basis represented by at least k × L computational qubits based on the state information acquired. The computational qubits 321 include at least L positional qubits corresponding to each of the candidate states, set for each of the k classifications, so that the computational basis is configured to represent for each classification which of the candidate states the particle takes. For example, the state information indicates the candidate states that each of the N particles can take. More specifically, the state information indicates, for example, the coordinates in state space of the possible states of each of the N particles. The position information in the description of the fourth process above can be considered an example of the above state information.

Each of the above information processing can constitute a technical idea by itself. For example, the fifth information processing can be used for any information processing that can be processed using quantum algorithms other than for obtaining calculation results related to physical phenomena such as nuclear configurations, e.g., calculation results of combinatorial optimization problems or traveling salesman problems.

Some of the processing of processor 23 may be converted into a computational algorithm that can be executed by a quantum computer and then have the quantum computer 3 execute the computational algorithm.

The quantum computer 3 may function as a quantum measurement apparatus. For example, in this case, the computational quantum circuit 5 is represented by unitary operations for initial state generation and free-time evolution.

Information processing apparatus 2 can be a classical or quantum computer or a combination of both.

The above information processing system 1 can be applied to various types of information processing related to quantum computation, such as quantum measurement and quantum communication.

The mode of the above embodiments is not limited to the information processing system 1, but may be an information processing method or an information processing program. The information processing method includes each step of the information processing system 1. The information processing program causes at least one computer to execute each step of the information processing system 1

The previous information processing method and the previous information processing program are not limited to those executed for ground state calculations, but may also be used to obtain low-energy states that are not ground states under realistic information processing constraints, such as calculation time and hardware configuration. For example, when an amorphous structure as a metastable state is output as a result of the above information processing for a system that constitutes a crystal as the ground state, the previous period information processing method or the previous period information processing program may be executed as a method to obtain the amorphous structure. In other words, the information processing system 1 may performs the identifying step to identify, based on the observation results for the first qubit in the generated correlated qubit state, among the configuration candidates of the nuclei, the configuration with relatively low energy eigenvalues for a given level instead of the lowest energy eigenvalue. This configuration facilitates the identification of various nuclear configurations, such as metastable structures.

The above information processing system 1, etc. may be provided in each of the following forms.

(1) An information processing system, comprising at least one processor configured to execute a program so as to perform each step of: an acquisition step of executing processing for acquiring substance information related to a substance including at least one atomic nucleus and at least one electron, the substance information including a plurality of configuration candidates of the atomic nucleus and interaction that acts on each of the atomic nucleus and the electron, the interaction including at least electron-nucleus interaction that acts between the atomic nucleus and the electron; an assignment step of executing processing for assigning each of the acquired configuration candidates of the atomic nucleus to at least one first qubit state, the first qubit state including at least one first qubit and being in an observable state by an observation operation to the first qubit; a superposition step of executing processing for generating a superposed state of a plurality of the first qubit states by executing a predetermined first quantum gate operation to the first qubit; an interaction step of executing processing for generating a correlated qubit state in which the first qubit and a second qubit are correlated by executing a second quantum gate operation including the acquired interaction to the first qubit representing the superposed state and the at least one second qubit representing an electronic state of the substance; and a specification step of executing processing for specifying a configuration having a relatively low minimum energy eigenvalue among the configuration candidates of the atomic nucleus based on an observation result on the first qubit in the generated correlated qubit state.

According to such a configuration, a plurality of configuration candidates of the atomic nuclei are represented by the first qubit. The first quantum gate operation allows the first qubit to represent the superposition state of a plurality of configuration candidates of the atomic nucleus. The electronic state represented by the second qubit interacts with such superposition states through the second quantum gate operation, so that the first and second qubits can represent the electronic state in the environment of the respective configuration candidates of the atomic nucleus. The probability of observing the nuclear configuration represented by the first qubit is higher when the energy of the entire system represented by the first and second qubits is lower. Therefore, by performing a second quantum gate operation that reduces the relative energy, the arrangement of particles with relatively low energy throughout the system is more likely to be observed from the first qubit. Therefore, a configuration with relatively low energy among the particle configurations is identified. Here, a state equivalent to that obtained by acting an electronic state on each of the configuration candidates of the nucleus individually can be generated by acting the electronic state on the superposition of the candidates Therefore, the calculation can be performed more efficiently than when performing calculations for each of a plurality of configuration candidates of the nucleus.

(2) The information processing system according to (1), wherein: the interaction step includes executing processing for generating a second qubit state representing an electronic state capable of converging to a ground state in a configuration of the atomic nucleus represented by the superposed state as a result of the interaction by executing the second quantum gate operation.

According to such a configuration, as the electronic state converges to the ground state, the energy of the entire system including the electronic state decreases. Thus, it is easier to identify the relatively low-energy configurations among the nuclear configurations.

(3) The information processing system according to (1) or (2), comprising the processor configured to execute a program so as to further perform each step of: an electron configuration step of executing processing for generating a superposed state of a reference electronic state for each of the configuration candidates of the atomic nucleus included in the generated superposed state by executing a reference quantum gate operation to the qubit including the generated superposed state, wherein the interaction step includes executing the second quantum gate operation to the qubit including the generated superposed state of the reference electronic state.

According to such a configuration, the calculation time to identify the low energy configuration can be reduced by setting an appropriate reference electronic state when realizing the energy state of the material by the second quantum gate operation.

(4) The information processing system according to any one of (1) to (3), wherein: the second quantum gate operation is configured to execute a predetermined ground state calculation method based on a first quantization form.

According to such a configuration, performing calculations based on the first quantization form makes it easier to obtain a picture of the nuclear configuration that is intuitively easy to grasp, compared to, for example, calculations based on the second quantization form, which makes it easier to interpret the calculation results.

(5) The information processing system according to any one of (1) to (4), wherein: the assignment step includes executing processing for assigning each of the configuration candidates of the atomic nucleus described as classical point charge to one of the first qubit states.

According to this configuration, the number of first qubits required to represent the arrangement of nuclei can be reduced compared to the case where the arrangement of nuclei is described including quantum fluctuations.

(6) The information processing system according to any one of (1) to (4), wherein: the substance information includes information on a reference position of the atomic nucleus, and the processor is configured to execute a program so as to further perform each step of: a candidate generation step of executing processing for generating a plurality of configuration candidates of the atomic nucleus by adding a predetermined displacement to the acquired reference position.

According to such a configuration, the time and effort required to input each of the configuration candidates of nucleus can be reduced.

(7) The information processing system according to any one of (1) to (6), wherein: the interaction further includes electron-electron interaction.

According to such a configuration, the electron-electron interaction has a larger contribution in the overall energy of the system than the electron-nucleus interaction. This makes it easier to identify the nuclear configuration with the lower energy, since the energy difference between the states produced by the second quantum gate operation is larger.

(8) The information processing system according to any one of (1) to (7), wherein: the substance comprises at least one molecular system.

According to such a configuration, the computational resources required to predict the shape of molecules such as proteins can be reduced, which have many degrees of freedom in the configuration of the nuclei.

(9) The information processing system according to any one of (1) to (8), wherein: the first quantum gate operation includes at least Hadamard gate operation.

According to such a configuration, the generation of superposition states of all initial states can be facilitated with a simple quantum operation, and thus prevents the calculation results from remaining at the local optimum solution due to the existence of non-superposed states.

(10) The information processing system according to any one of (1) to (9), wherein: the first quantum gate operation is configured to act on a computational qubit including the first qubit and the second qubit so that the computational qubit takes on an eigenstate of a predetermined initial Hamiltonian, and the second quantum gate operation is a gate operation corresponding to a virtual Hamiltonian that gives time evolution approximately adiabatically from the initial Hamiltonian to the interaction using at least one real-time evolution operator.

According to such a configuration, the quantum circuit in identifying the optimal arrangement is simplified, especially in a non-degenerate system. Thus, it is easier to identify the optimal placement in a non-degenerate system.

(11) The information processing system according to (10), comprising the processor configured to execute a program so as to further perform each step of: a parameter determination step of generating a virtual state by allowing the virtual Hamiltonian including a time width of the time evolution as a parameter to act on a superposed state of the first qubit state, and determining the parameter by minimizing an energy expected value of the virtual state for a final state of the virtual Hamiltonian due to time evolution.

According to such a configuration, calculation results with the desired accuracy can be obtained in a short time.

(12) The information processing system according to any one of (1) to (11), wherein: the specification step includes executing processing for specifying, from among the configuration candidates of the atomic nucleus, a configuration in which an energy eigenvalue of a predetermined level is relatively low, instead of the minimum energy eigenvalue, based on an observation result on the first qubit in the generated correlated qubit state.

This configuration facilitates the identification of various nuclear configurations, such as metastable structures.

(13) An information processing system, comprising at least one processor configured to execute a program so as to perform each step of: an acquisition step of executing processing for acquiring substance information related to a substance including at least one particle, the substance information including a plurality of arrangement candidates of the particle and interaction that acts on the particle, the interaction including at least particle-particle interaction that acts between the particles; an assignment step of assigning each of the acquired arrangement candidates of the particle to at least one first qubit state, the first qubit state being in a state that includes at least one first qubit and is observable by an observation operation to the first qubit; a superposition step of executing processing for generating a superposed state of a plurality of the first qubit states by executing a predetermined first quantum gate operation to the first qubit; an interaction step of executing processing for generating a correlated qubit state in which the first qubits are correlated by executing a second quantum gate operation including the acquired interaction to the first qubit representing the superposed state; and a specification step of executing processing for specifying an arrangement having a relatively low energy among the arrangement candidates of the particle based on an observation result on the first qubit in the generated correlated qubit state.

According to such a configuration, a plurality of arrangement candidates of the particle is represented by the first qubit. The first qubit can then represent the superposition state of a plurality of arrangement candidates of the particle by the first quantum gate operation. The probability of observing the particle configuration represented by the first qubit is higher when the energy of the entire system represented by the first qubit is lower. Therefore, by performing a second quantum gate operation that reduces the relative energy, the arrangement of particles with relatively low energy throughout the system is more easily observed from the first qubit. Therefore, a configuration with relatively low energy among the particle configurations is identified. Therefore, the calculation can be performed more efficiently than if the energy were calculated for each of multiple configuration candidates of the particle individually.

(14) An information processing system, comprising at least one processor configured to execute a program so as to perform each step of: an acquisition step of acquiring position information related to L arrangement candidates in a certain space and classification information related to k types of classification of N particles to be arranged in the arrangement candidates; and a basis generation step of generating a computational basis represented by at least k x L computational qubits based on the acquired position information, the computational qubits including at least L position qubits corresponding to each of the arrangement candidates set for each of the k types of classification, so that the computational basis is configured to represent, for each of the classification, which of the arrangement candidates the particle is arranged in.

According to such a configuration, it is possible to reduces the number of computational qubits required to represent multiple particle states.

(15) The information processing system according to (14), wherein: the acquisition step further includes acquiring information related to interaction between the particles to be arranged in the arrangement candidates and information related to a number of the particles for each of the classification, the interaction is determined by at least a positional relationship between the particles and classification of the particles, the processor is configured to execute a program so as to further perform each step of: a generation step of generating an objective function based on the computational basis and the interaction, the objective function includes a first factor and a second factor, the first factor corresponds to Hamiltonian in a system defined by the particles, and the second factor is configured to output a larger value when a first number representing a number of the particles of a certain classification represented by the computational qubits is different from a second number representing the number of the particles acquired, compared to when the first number matches the second number.

According to such a configuration, the possibility that a computational basis corresponding to the configuration that has the different number of the particles from that of the assumed system is computed as the optimal one can be reduced when estimating the optimal configuration of the particles.

(16) The information processing system according to (15), wherein: the second factor is represented by an even function of a difference between the first number and the second number.

According to such a configuration, cases in which the number of particles is less than or greater than the assumed system can be treated equivalently, thus reducing bias in the calculation.

(17) The information processing system according to (15) or (16), wherein: the objective function is represented by an Ising-type function or in a QUBO format.

According to such a configuration, the execution of calculations in quantum computation using a quantum annealing machine can be realized.

(18) The information processing system according to any one of (15) to (17), comprising the processor configured to execute a program so as to further perform each step of: a superposition step of executing processing for generating a first superposed state in which a state corresponding to the computational basis has been superposed by executing a first quantum operation to the computational qubits; an interaction step of executing processing for generating a second superposed state in which interaction that acts at least between the particles has been reflected in the first superposed state by executing a second quantum operation corresponding to the objective function to the first superposed state; and a specification step of executing processing for specifying an arrangement having a relatively low energy among the arrangement candidates of the particles based on an observation result on the computational qubits in the generated second superposed state.

According to such a configuration, the calculation can be performed more efficiently than when calculating the energy for each of multiple configuration candidates of the particles individually.

(19) The information processing system according to any one of (14) to (18), wherein: each of the particles is an atomic nucleus that constitutes a substance.

According to such a configuration, the simulation of material can be made easily.

(20) The information processing system according to any one of (14) to (19), wherein: each of the particles is a classical point particle.

According to such a configuration, the number of computational qubits required for a calculation can be reduced compared to the case of using a particle image spread over quantum space.

(21) The information processing system according to any one of (14) to (20), wherein: the classification includes atomic species of a particle.

According to such a configuration, the increase in the number of computational qubits due to the increase in the number of particles can be suppressed, since at least L position qubits can be used to represent the arrangement candidates for each atomic species.

(22) The information processing system according to any one of (14) to (21), wherein: the basis generation step includes generating a computational basis configured by n x k x L computational qubits, where n is an integer equal to or greater than 2, so that the computational basis is further configured to represent a case in which a plurality of the particles is present in the same arrangement candidate.

According to such a configuration, identifying the optimal arrangement of particles in more diverse systems becomes easier.

(23) An information processing system, comprising at least one processor configured to execute a program so as to perform each step of: an acquisition step of acquiring state information related to L state candidates in a certain state space and classification information related to k types of classification of N particles, each of the particles taking any one of the state candidates; and a basis generation step of generating a computational basis represented by at least k x L computational qubits based on the acquired state information, the computational qubits including at least L position qubits corresponding to each of the state candidates set for each of the k types of classification, so that the computational basis is configured to represent, for each of the classification, which state of the state candidates the particle takes.

According to such a configuration, it is possible to reduces the number of computational qubits required to represent multiple particle states.

(24) An information processing system, comprising at least one processor configured to execute a program so as to perform each step of: an acquisition step of acquiring information related to a plurality of specific positions, which is a position where a particle can be arranged in a certain space; and an assignment step of assigning a computational qubit group including at least one computational qubit to each of the specific positions, the computational qubit group being configured so that at least a first state and a second state become observable states, the first state indicating presence of the particle at the corresponding specific position, the second state indicating absence of the particle at the corresponding specific position.

Such a configuration enhances the interpretability of quantum calculations because the state of the particle in a specific position is represented as the state of the computational qubit.

(25) The information processing system according to (24), wherein: the acquisition step further includes acquiring classification information representing classification of the particle, and the assignment step further includes assigning the at least one computational qubit to each of the specific positions for each of the classification of the particle.

According to this configuration, the state of a complex system in which particles of multiple classifications are mixed can be represented with a smaller number of computational qubits.

(26) The information processing system according to (25), wherein: the classification of the particle includes at least atomic species of the particle.

According to this configuration, the state of a complex system with multiple atomic species can be represented with a smaller number of computational qubits.

(27) The information processing system according to any one of (24) to (26), wherein: the computational qubit group includes two or more computational qubits and is configured so that at least one qubit state different from the first state and the second state is observable, and the qubit state is each associated with the number of particles present at the corresponding specific position.

According to this configuration, the interpretability of quantum calculations for more diverse systems can be improved, because the state in which multiple particles exist in the same specific position can be assigned to the state of a computational qubit, as in boson systems, for example.

(28) An Information processing method, comprising each of the steps of the information processing system according to any one of (1) to (27).

(29) An information processing program, configured to allow at least one computer to execute each of the steps of the information processing system according to any one of (1) to (27).

Of course, it is not limited to that.

Finally, although various embodiments of the present invention have been described, these are presented as examples and are not intended to limit the scope of the present invention. The novel embodiments may be implemented in various other forms, and various omissions, substitutions, and modifications may be made within the scope of invention without departing from the gist of the present invention. The embodiments or modifications thereof are included in the scope and gist of the present invention, as well as within the scope of the invention and equivalents thereof recited in the claims.

[Description of Sign]

1: Information processing system
2: Information processing apparatus
3: Quantum computer
4: User terminal
5: Computational quantum circuits
20: Communication bus
21: Communication unit
22: Storage unit
23: Processor
231: Acquisition unit
232: Candidate Generation unit
233: Assignment unit
234: Quantum manipulation unit
235: Identification unit
30: Communication bus
31: Communication unit
32: Quantum Memory
320: Qubit
321: Computational qubit
321n: First qubit
321e: Second qubit
322: Ancillary bits
323: Environmental bit
33: Quantum Processor
40: Communication bus
41: Communication unit
42: Memory
43: Processor
44: Display unit
45: Input unit
B1: Bar graph
C__PITE: search quantum circuit
L1: Line graph
Q: Quantum amplitude amplification circuit
QC1: Computational quantum circuit
QC10: Input generation circuit
QC11: First quantum gate operation
QC12: Reference quantum gate operation
QC13: Second quantum gate operation
QC131: Unit quantum circuit
QC14: Observation operation
S_0: Zero reflection
S_$\varphi$: Reflection operator

S_x: Oracle
U_ME: State Generation Circuit
U_PITE: Quantum Circuit
U_ref: Reference circuit

**Claims**

1. An information processing system, comprising at least one processor configured to execute a program so as to perform each step of:

   an acquisition step of executing processing for acquiring substance information related to a substance including at least one atomic nucleus and at least one electron,

      the substance information including a plurality of configuration candidates of the atomic nucleus and interaction that acts on each of the atomic nucleus and the electron,
      the interaction including at least electron-nucleus interaction that acts between the atomic nucleus and the electron;

      an assignment step of executing processing for assigning each of the acquired configuration candidates of the atomic nucleus to at least one first qubit state, the first qubit state including at least one first qubit and being in an observable state by an observation operation to the first qubit;
      a superposition step of executing processing for generating a superposed state of a plurality of the first qubit states by executing a predetermined first quantum gate operation to the first qubit;
      an interaction step of executing processing for generating a correlated qubit state in which the first qubit and a second qubit are correlated by executing a second quantum gate operation including the acquired interaction to the first qubit representing the superposed state and the at least one second qubit representing an electronic state of the substance; and
      a specification step of executing processing for specifying a configuration having a relatively low minimum energy eigenvalue among the configuration candidates of the atomic nucleus based on an observation result on the first qubit in the generated correlated qubit state.

2. The information processing system according to claim 1, wherein:
   the interaction step includes executing processing for generating a second qubit state representing an electronic state capable of converging to a ground state in a configuration of the atomic nucleus represented by the superposed state as a result of the interaction by executing the second quantum gate operation.

3. The information processing system according to claim 1 or 2, comprising the processor configured to execute a program so as to further perform each step of:

   an electron configuration step of executing processing for generating a superposed state of a reference electronic state for each of the configuration candidates of the atomic nucleus included in the generated superposed state by executing a reference quantum gate operation to the qubit including the generated superposed state, wherein the interaction step includes executing the second quantum gate operation to the qubit including the generated superposed state of the reference electronic state.

4. The information processing system according to any one of claims 1 to 3, wherein:
   the second quantum gate operation is configured to execute a predetermined ground state calculation method based on a first quantization form.

5. The information processing system according to any one of claims 1 to 4, wherein:
   the assignment step includes executing processing for assigning each of the configuration candidates of the atomic nucleus described as classical point charge to one of the first qubit states.

6. The information processing system according to any one of claims 1 to 4, wherein:

   the substance information includes information on a reference position of the atomic nucleus, and
   the processor is configured to execute a program so as to further perform each step of:

a candidate generation step of executing processing for generating a plurality of configuration candidates of the atomic nucleus by adding a predetermined displacement to the acquired reference position.

**7.** The information processing system according to any one of claims 1 to 6, wherein:
the interaction further includes electron-electron interaction.

**8.** The information processing system according to any one of claims 1 to 7, wherein:
the substance comprises at least one molecular system.

**9.** The information processing system according to any one of claims 1 to 8, wherein:
the first quantum gate operation includes at least Hadamard gate operation.

**10.** The information processing system according to any one of claims 1 to 9, wherein:

the first quantum gate operation is configured to act on a computational qubit including the first qubit and the second qubit so that the computational qubit takes on an eigenstate of a predetermined initial Hamiltonian, and the second quantum gate operation is a gate operation corresponding to a virtual Hamiltonian that gives time evolution approximately adiabatically from the initial Hamiltonian to the interaction using at least one real-time evolution operator.

**11.** The information processing system according to claim 10, comprising the processor configured to execute a program so as to further perform each step of:
a parameter determination step of

generating a virtual state by allowing the virtual Hamiltonian including a time width of the time evolution as a parameter to act on a superposed state of the first qubit state, and
determining the parameter by minimizing an energy expected value of the virtual state for a final state of the virtual Hamiltonian due to time evolution.

**12.** The information processing system according to any one of claims 1 to 11, wherein:
the specification step includes executing processing for specifying, from among the configuration candidates of the atomic nucleus, a configuration in which an energy eigenvalue of a predetermined level is relatively low, instead of the minimum energy eigenvalue, based on an observation result on the first qubit in the generated correlated qubit state.

**13.** An information processing system, comprising at least one processor configured to execute a program so as to perform each step of:

an acquisition step of executing processing for acquiring substance information related to a substance including at least one particle, the substance information including a plurality of arrangement candidates of the particle and interaction that acts on the particle, the interaction including at least particle-particle interaction that acts between the particles;
an assignment step of assigning each of the acquired arrangement candidates of the particle to at least one first qubit state, the first qubit state being in a state that includes at least one first qubit and is observable by an observation operation to the first qubit;
a superposition step of executing processing for generating a superposed state of a plurality of the first qubit states by executing a predetermined first quantum gate operation to the first qubit;
an interaction step of executing processing for generating a correlated qubit state in which the first qubits are correlated by executing a second quantum gate operation including the acquired interaction to the first qubit representing the superposed state; and
a specification step of executing processing for specifying an arrangement having a relatively low energy among the arrangement candidates of the particle based on an observation result on the first qubit in the generated correlated qubit state.

**14.** An information processing system, comprising at least one processor configured to execute a program so as to perform each step of:

an acquisition step of acquiring position information related to L arrangement candidates in a certain space and classification information related to k types of classification of N particles to be arranged in the arrangement candidates; and

a basis generation step of generating a computational basis represented by at least k x L computational qubits based on the acquired position information, the computational qubits including at least L position qubits corresponding to each of the arrangement candidates set for each of the k types of classification, so that the computational basis is configured to represent, for each of the classification, which of the arrangement candidates the particle is arranged in.

15. The information processing system according to claim 14, wherein:

the acquisition step further includes acquiring information related to interaction between the particles to be arranged in the arrangement candidates and information related to a number of the particles for each of the classification, the interaction is determined by at least a positional relationship between the particles and classification of the particles,

the processor is configured to execute a program so as to further perform each step of:

a generation step of generating an objective function based on the computational basis and the interaction,

the objective function includes a first factor and a second factor,
the first factor corresponds to Hamiltonian in a system defined by the particles, and
the second factor is configured to output a larger value when a first number representing a number of the particles of a certain classification represented by the computational qubits is different from a second number representing the number of the particles acquired, compared to when the first number matches the second number.

16. The information processing system according to claim 15, wherein:
the second factor is represented by an even function of a difference between the first number and the second number.

17. The information processing system according to claim 15 or 16, wherein:
the objective function is represented by an Ising-type function or in a QUBO format.

18. The information processing system according to any one of claims 15 to 17, comprising the processor configured to execute a program so as to further perform each step of:

a superposition step of executing processing for generating a first superposed state in which a state corresponding to the computational basis has been superposed by executing a first quantum operation to the computational qubits;

an interaction step of executing processing for generating a second superposed state in which interaction that acts at least between the particles has been reflected in the first superposed state by executing a second quantum operation corresponding to the objective function to the first superposed state; and

a specification step of executing processing for specifying an arrangement having a relatively low energy among the arrangement candidates of the particles based on an observation result on the computational qubits in the generated second superposed state.

19. The information processing system according to any one of claims 14 to 18, wherein:
each of the particles is an atomic nucleus that constitutes a substance.

20. The information processing system according to any one of claims 14 to 19, wherein:
each of the particles is a classical point particle.

21. The information processing system according to any one of claims 14 to 20, wherein:
the classification includes atomic species of a particle.

22. The information processing system according to any one of claims 14 to 21, wherein:
the basis generation step includes generating a computational basis configured by n x k x L computational qubits, where n is an integer equal to or greater than 2, so that the computational basis is further configured to represent a case in which a plurality of the particles is present in the same arrangement candidate.

23. An information processing system, comprising at least one processor configured to execute a program so as to perform each step of:

an acquisition step of acquiring state information related to L state candidates in a certain state space and classification information related to k types of classification of N particles, each of the particles taking any one of the state candidates; and

a basis generation step of generating a computational basis represented by at least k x L computational qubits based on the acquired state information, the computational qubits including at least L position qubits corresponding to each of the state candidates set for each of the k types of classification, so that the computational basis is configured to represent, for each of the classification, which state of the state candidates the particle takes.

24. An information processing system, comprising at least one processor configured to execute a program so as to perform each step of:

an acquisition step of acquiring information related to a plurality of specific positions, which is a position where a particle can be arranged in a certain space; and

an assignment step of assigning a computational qubit group including at least one computational qubit to each of the specific positions,

the computational qubit group being configured so that at least a first state and a second state become observable states,

the first state indicating presence of the particle at the corresponding specific position,

the second state indicating absence of the particle at the corresponding specific position.

25. The information processing system according to claim 24, wherein:

the acquisition step further includes acquiring classification information representing classification of the particle, and

the assignment step further includes assigning the at least one computational qubit to each of the specific positions for each of the classification of the particle.

26. The information processing system according to claim 25, wherein:
the classification of the particle includes at least atomic species of the particle.

27. The information processing system according to any one of claims 24 to 26, wherein:

the computational qubit group includes two or more computational qubits and is configured so that at least one qubit state different from the first state and the second state is observable, and

the qubit state is each associated with the number of particles present at the corresponding specific position.

28. An Information processing method, comprising each of the steps of the information processing system according to any one of claims 1 to 27.

29. An information processing program, configured to allow at least one computer to execute each of the steps of the information processing system according to any one of claims 1 to 27.

FIG. 1

FIG. 2

FIG. 3

41                    42                    43              4

COMMUNICATION
UNIT              MEMORY           PROCESSOR

DISPLAY
UNIT              INPUT UNIT

USER
TERMINAL

40

44                    45

FIG. 4

23

PROCESSOR

ACQUISITION UNIT — 231

CANDIDATE GENERATION
UNIT — 232

ASSIGNMENT UNIT — 233

QUANTUM
MANIPULATION UNIT — 234

IDENTIFICATION UNIT — 235

FIG. 5

USER TERMINAL 4    INFORMATION PROCESSING  QUANTUM COMPUTER 3
APPARATUS 2

A1
Send substance
information

A2
Acquire substance
information

A3
Candidate generation
process

A4
Assignment process

A5
Quantum circuit
generation process

A6
Send the quantum circuit

A8
Acquire observation
results

A7
Perform a quantum
operation based on the
quantum circuit

A9
Aggregate observation
results

[Termination
condition
unachieved]

[Termination condition
achieved]

A11
Display the configuration
of the nucleus

A10
Identify a configuration of
the nucleus

FIG. 6

FIG. 7

$$\text{Nuclei } |0\rangle_{3n_{\text{nucl}}n_{qn}} \quad /\!\!-\!\!\boxed{U_{\text{guess}}}\!-\!\sum_{\boldsymbol{J}} \sqrt{w_{\text{guess},\boldsymbol{J}}}|\boldsymbol{J}\rangle_{3n_{\text{nucl}}n_{qn}}$$

321n

QC11

FIG.8

321n

QC12

$$\text{Nuclei } |\boldsymbol{J}\rangle_{3n_{\mathrm{nucl}}n_{\mathrm{qn}}} \quad |\boldsymbol{J}\rangle_{3n_{\mathrm{nucl}}n_{\mathrm{qn}}}$$

$$\text{Electrons } |0\rangle_{3n_e n_{qe}} \quad U_{\mathrm{ref}} \quad |\psi_{\mathrm{ref}}[\boldsymbol{J}]\rangle$$

321e

FIG. 9

FIG. 10

EP 4 597 505 A1

FIG. 11

FIG. 12

EP 4 597 505 A1

FIG. 13

FIG. 14

EP 4 597 505 A1

FIG. 15

57

FIG. 16

FIG. 17

FIG. 18

USER TERMINAL 4    INFORMATION PROCESSING  QUANTUM COMPUTER 3
                         APPARATUS 2

FIG. 19

61

FIG. 20

EP 4 597 505 A1

FIG. 21

FIG. 22

FIG. 23

FIG. 24

FIG. 25

FIG. 26

```
                    ┌─────────────┐
                    │    Start    │
                    └─────────────┘
                          │
    S1                    ▼
          ┌───────────────────────────────┐
          │   Set the initial Hamiltonian  │
          └───────────────────────────────┘
    S2                    │
                          ▼
          ┌───────────────────────────────┐
          │ Set the ground state corresponding │
          │     to the initial Hamiltonian    │
          └───────────────────────────────┘
    S3                    │
                          ▼
          ┌───────────────────────────────┐
          │   Generate the input generation  │
          │             circuit             │
          └───────────────────────────────┘
    S4                    │
                          ▼
          ┌───────────────────────────────┐
          │  Generate the second quantum gate │
          │            operation            │
          └───────────────────────────────┘
    S5                    │
                          ▼
          ┌───────────────────────────────┐
          │   Generate the computational     │
          │        quantum circuit          │
          └───────────────────────────────┘
                          │
                          ▼
                    ┌─────────────┐
                    │     End     │
                    └─────────────┘
```

FIG. 27

FIG. 28

FIG. 29

FIG. 30

FIG. 31

FIG. 32

Start

S11

Set the initial Hamiltonian

S12

Set the ground state corresponding to the initial Hamiltonian

S13

Set the virtual state

S14

Set the computational quantum circuit

S15

Compute the energy expectation value of the virtual state based on the computational quantum circuit

S16

Identify the parameters that give the lowest energy expectation value

End

FIG. 33

FIG. 34

**EP 4 597 505 A1**

## INTERNATIONAL SEARCH REPORT

| International application No. |
| --- |
| **PCT/JP2023/027741** |

| A. | CLASSIFICATION OF SUBJECT MATTER |
| --- | --- |

*G16C 10/00*(2019.01)i; *G06N 10/40*(2022.01)i; *G06N 10/80*(2022.01)i; *G06N 99/00*(2019.01)i
FI: G16C10/00; G06N10/40; G06N10/80; G06N99/00 180

According to International Patent Classification (IPC) or to both national classification and IPC

| B. | FIELDS SEARCHED |
| --- | --- |

Minimum documentation searched (classification system followed by classification symbols)

G16C10/00-99/00; G06N10/40; G06N10/80; G06N99/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | 笠松秀輔, 結晶材料中の不規則原子配置のモンテカルロサンプリング, 日本神経回路学会誌 [online], vol. 28, no. 1, 05 April 2021, pp. 12-19, [retrieved on 16 August 2023], Retrieved from the Internet: <URL: https://doi.org/10.3902/jnns.28.12> (KASAMATSU, Shusuke. Monte Carlo Sampling of Configuration Disorder in Crystalline Materials. Journal of the Japanese Neural Network Society.)<br>entire text, all drawings | 1-13 |
| A | JP 2021-504831 A (1QB INFORMATION TECHNOLOGIES INC.) 15 February 2021 (2021-02-15)<br>entire text, all drawings | 1-13 |
| A | JP 2021-033768 A (FUJITSU LTD.) 01 March 2021 (2021-03-01)<br>entire text, all drawings | 1-13 |
| A | 西村 治道, 量子計算の基礎, 情報処理 , 15 June 2014, vol. 55, no. 7, pp. 682-688 non-official translation (NISHIMURA, Harumichi. Fundamentals of Quantum Computing. Information Processing.)<br>pp. 682-688 | 14-23 |

| ✓ Further documents are listed in the continuation of Box C. | ✓ See patent family annex. |
| --- | --- |

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **02 October 2023** | **10 October 2023** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

# EP 4 597 505 A1

## INTERNATIONAL SEARCH REPORT

<table>
<tr><td colspan="2"></td><td>International application No.<br>**PCT/JP2023/027741**</td></tr>
</table>

### C. DOCUMENTS CONSIDERED TO BE RELEVANT

| Category* | Citation of document, with indication, where appropriate, of the relevant passages | Relevant to claim No. |
|---|---|---|
| X | SINGH, Satya Pal, The Ising Model: Brief Introduction and Its Application, Metastable, Spintronics Materials and Mechanics of Deformable Bodies [online], 24 February 2020, pp. 1-18, [retrieved on 02 October 2023], Retrieved from the Internet: <URL: https://doi.org/10.5772/intechopen.90875> <br> pp. 2-5 | 24, 28-29 |
| A | pp. 1-18 | 25-27 |

Form PCT/ISA/210 (second sheet) (January 2015)

**INTERNATIONAL SEARCH REPORT**
Information on patent family members

International application No.

**PCT/JP2023/027741**

| Patent document cited in search report | | | Publication date (day/month/year) | Patent family member(s) | | | Publication date (day/month/year) |
|---|---|---|---|---|---|---|---|
| JP | 2021-504831 | A | 15 February 2021 | US | 2020/0364601 | A1 | |
| | | | | entire text, all drawings | | | |
| | | | | WO | 2019/104440 | A1 | |
| | | | | EP | 3718060 | A1 | |
| | | | | CN | 111656375 | A | |
| | | | | CA | 3082937 | A1 | |
| JP | 2021-033768 | A | 01 March 2021 | US | 2021/0064799 | A1 | |
| | | | | entire text, all drawings | | | |
| | | | | EP | 3786963 | A1 | |
| | | | | CN | 112446127 | A | |

Form PCT/ISA/210 (patent family annex) (January 2015)

**EP 4 597 505 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008052308 A

**Non-patent literature cited in the description**

- **P. J. OLLITRAULT** ; **G. MAZZOLA** ; **I. TAVERNELLI**. Nonadiabatic molecular quantum dynamics with quantum computers. *Phys. Rev. Lett.*, 2020, vol. 125, 260511

- **G. BENENTI** ; **G. STRINI**. Quantum simulation of the single-particle Schroedinger equation. *American Journal of Physics*, 2008, vol. 76, 657
- **S. MCARDLE et al.** Variational ansatz-based quantum simulation of imaginary time evolution. *npj Quantum Information*, 2019, vol. 5, 75